# EUROPEAN PATENT SPECIFICATION

(11) **EP 0 638 807 B1**
(45) Date of publication and mention of the grant of the patent: **17.07.2002**
(21) Application number: 94117589.5
(22) Date of filing: 21.09.1988
(51) Int. Cl.: G01N 33/542, G01N 33/533, C12Q 1/68, G01N 33/58, G01N 33/532

(54) **Protection assay**
Schutztest
Essai de protection

(30) Priority: 21.09.1987 US 99392
(43) Date of publication of application: 15.02.1995
(62) Divisional of application: 88308767.8
(73) Proprietor: Gen-Probe Incorporated, San Diego, CA 92121 (US)
(72) Inventor: Arnold, Lyle John, Poway, California 92064 (US); Nelson, Norman C., San Diego, California 92111 (US)
(74) Representative: Sexton, Jane Helen

(56) References cited:
- EP-A- 0 082 636
- EP-A- 0 131 830
- EP-A- 0 212 951
- CLINICAL CHEMISTRY, vol.35, no.8, 1989, WINSTON-SALEM NC USA pages 1588 - 1594 L.J. ARNOLD ET AL. 'Assay formats involving acridine-ester-labelled DNA probes.'

## Description

### Background

This invention is in the art of diagnostic procedures and techniques for detecting and quantitating minute amounts of organic compounds.

More particularly, this invention relates to homogeneous diagnostic assays in which there is a difference in the stability of the label in its bound, as opposed to its unbound forms. This invention relates to the construction of environments for diagnostic assay systems in which a label is differentially degraded in either its complexed or bound form as compared to its uncomplexed or unbound form.

### Background of the Invention

Diagnostic assays are a common analytical technique for detecting, locating or quantifying biological substances by employing labelled binding reagents. The presence of the labeled reagent can then be detected using a variety of known methods. This invention can be applied to all known diagnostic assay formats, including, without limitation, direct binding and competition assays and sequential saturation. One particular type of diagnostic assay is the nucleic acid hybridization assay. Hybridization assay systems are based on the fact that single stranded nucleic acids (DNA or RNA) will hybridize or recombine, under appropriate circumstances, with complementary single stranded nucleic acids. By labelling the complementary probe nucleic acid with a readily detectable label, it is possible to detect the presence of the target polynucleotide sequence of interest in a test sample containing single stranded nucleic acid sequences.

Assay systems may broadly be characterized as heterogeneous or homogeneous. The term "heterogeneous" as applied to assay systems means those specific binding assays which require a separation of the labelled from unlabeled substance to be detected. Homogeneous assays systems, on the other hand, do not involve any physical separation steps. Because homogeneous assay systems involve a minimum number of handling steps they are generally more convenient and easier to use than heterogeneous assay systems.

For example, a typical sandwich immunoassay may involve incubating an immobilized antibody with a test medium. Antigens, if in the medium, will bind to the antibody. After incubation, unbound antigen is removed in a separation step. After a second, or simultaneous incubation with a solution of labeled antibody, the bound antigen becomes "sandwiched" between the immobilized antibody and the labelled antibody. After a second separation step, the amount of labeled antibody can be determined as a measure of the antigen in the medium. This system is time consuming because it involves a series of incubation and separation steps.

A focus of effort of the prior art in diagnostic assays has been directed to developing homogenous assays and labels which can discriminate between minor differences in the amount of bound, as opposed to unbound substances of interest. One of the objects of the present invention is an assay system in which the label itself undergoes a detectable change, which may be, for example, in its ability to chemiluminesce, when it is in its bound, as opposed to when it is unbound. Another object of the present invention is an assay system in which the label is substantially degraded or destroyed in either its bound or unbound form, thereby providing a ready means for identifying and quantitating a reaction of interest.

It is an object of the present invention to disclose an improved method for sensitively detecting analytes using a homogeneous assay format. It is also an object of this invention to provide improved methods for increasing the sensitivity of assays which involve separation by combining the homogeneous method disclosed herein with other separation methods to reduce non-specific background. The principle of the invention disclosed here is based upon the differential stability of a label to chemical or biochemical reagents. Whenever certain labels are conjugated to binding partners, we have found that the stability of said labels are or may be altered when said binding partner is bound to a binding substance of said binding partner. It is also the object of this invention to disclose a method by which said differential label stability may be employed for the sensitive detection of an analyte employing a homogeneous diagnostic assay system. Yet another object of the present invention is to disclose the use of chemiluminescent acridinium ester labeled DNA probes in said homogeneous diagnostic assays for sensitively detecting the presence of complementary target polynucleotide sequences.

### Description of the Prior Art

There are a variety of homogeneous assays in the prior art which vary in complexity. In some systems, for example, the label is a catalyst which can participate in a chemical reaction in the presence of other components, for example, enzymes, coenzymes and cofactors. In other, albeit related systems, the label is a substrate for a chemical or biochemical reaction. In these systems, the generation of a specific readily detectable substance, for example, glucose, lactate or alcohol is used to monitor and measure the target reaction. In other assay systems, the analyte possesses unique physical properties which allow it to be directly detected. Examples of these types of labels include metals, hemoglobin and chlorophyll.

Still other homogenous assay systems are based on enzyme coupled specific binding reactions, wherein the analyte is a ligand for a specific binding reaction. When the analyte is present, it undergoes a specific binding reaction with a conjugate which is comprised of a specific binding partner and a labelling substance. Either concurrently or subsequently, other substituents are added which interact with the label. The activity of the label is different when the conjugate of which the label is a component, is in a complexed form verses an uncomplexed form. Such systems have typically used enzymes as the labelling reagent and substrates which produce a colorimetric, fluorimetric, or chemiluminescent end point. Examples of homogeneous enzyme immunoassays include U.S. Patent Nos. 3,654,090, 3,817,837 and 4,190,496. Other examples of homogeneous assays involving the use of chromophores which make up fluorescer/quencher pairs may be found in U.S. Patent Nos. 4,199,559, 4,174,384 and 4,318,707. In some systems, however, the label has been a substance other than an enzyme, for example, vitamins, NAD, FAD or biotin, which nevertheless can be coupled to a "monitoring" reaction. An example of this type is U.S. Patent No. 4,383,031. In these systems, the monitoring reaction is based upon a structural change which modifies the activity of the label.

Other homogenous assay systems involve the technique of polarization fluorescence. Here, the analyte competes with a low molecular weight fluorescent conjugate for binding to a high molecular weight specific binding partner. Since the polarization of the fluorescence changes when the small molecule is displaced from the surface of the large molecule, it is possible to determine the amount of analyte in solution. An example of this type of assay system is U.S. Patent No. 4,668,640.

Yet another type of homogenous assay system involves non-radiative energy transfer. In these systems, the absorption of light from one molecule to another when the two molecules come into close proximity is used as the monitoring reaction. Generally, these methods have been described in two ways. In one method, the first molecule is chemiluminescent and upon excitation a portion of the electromagnetic energy generated is transferred to a second "absorber" molecule which must be in close proximity. If the energy is absorbed by the second molecule and emitted at a different wavelength, a portion of the light will show a spectral shift proportional to the number of chemiluminescent molecules in close proximity to absorber molecules.

In another type of non-radiative energy assay system, the first molecule is fluorescent and serves as an "absorber" of external light. In the presence of a second fluorescent molecule a portion of the energy is transferred and light is emitted at a different wavelength. The emitted light will show a spectral shift proportional to the number of "absorber" and "emitter" molecules in close proximity to each other.

A different type of double probe assay system is seen in U.S. Patent No. 4,670,379. The first probe is labeled with a catalyst; the second with an apoluminescer. Both probes target neighboring areas on the target nucleic acid sequence. Once both probes are hybridized together with the target, the substrate is added. The catalyst converts the substrate to a transformation radical which, in turn, converts the apoluminescer on the second probe to a luminescer. This occurs only if hybridization has taken place. Employing these principles, assays have been developed based upon two labelled substances simultaneously binding a common analyte.

A specific example of this type of energy transfer assay system is Elazar et al., European Patent Application No. 85105130.0, Publication No. 0159719 published October 30, 1985, which discloses using two single stranded nucleic acid probes which are complementary to the same or opposite strands of the target genetic material. Each probe is labeled with a moiety capable of producing a signal only when the two labels are brought together. Unlike the invention herein described, Elazar et al. involves the formation and detection of double hybrid or multihybrids. Similarly, Heller et al., European Patent Application No. 82303699.1, Publication No. 0070685 dated January 26, 1983 and related Morrison et al., European Patent Application No. 82303700.7, Publication No. 0070686 of the same date disclose homogenous assay systems using two luminescer probes. At least one of the light labels is of the absorber/emitter type so that when the two probes are hybridized to the target, energy transfer occurs between the two labels. This causes the absorber/emitter to re-emit at a different wavelength. The second emission detects hybridization. An antigen assay of this type is disclosed in Morrisson et al. supra.

Most biological substances can not be readily detected directly at any reasonable level of sensitivity and require a binding reaction of some type. As evident from the foregoing discussion, the prior art is based on the detection of minor attenuations between bound and unbound label. The prior art systems are not capable of significant discrimination between bound and unbound label. Such assays have been found useful for detecting analytes which are present only at high concentration, for example in the monitoring of various drugs in blood and urine.

There is a need in the area of clinical diagnostics for a direct detection homogenous assay which is based on the ability of two binding partners to modify the stability of the label, for example, resulting in selective removal or destruction of label in either the bound or unbound form. While Hirschfield, in Florescence Background Discrimination by Prebleaching J. Histochemistry and Cytochemistry, 27/1, 96-101 (1979) describes a somewhat related electrooptics technique involving photochemical bleaching which may destroy label molecules, or at least their fluorescence. The invention disclosed therein does not teach or suggest use of photochemical bleaching or any other technique for selective removal or destruction of a label in a diagnostic assay. The subject invention fulfills a present need in diagnostic assays as it is orders of magnitude more sensitive than the prior art. The range of sensitivity of the prior art is no better than the 10⁻¹³ mole range, while the present invention is sensitive in the 10⁻¹⁶ mole range.

### Summary of the Invention

Briefly, this invention comprises diagnostic assays and methods. The method may be used to detect an analyte in a medium, said analyte being part of a specific binding pair. When the medium suspected of containing the analyte is combined with a binding partner, a label substance attached to the binding partner is capable of undergoing a detectable change in stability or differential degradation whenever the analyte binds to the specific binding partner. In a specific embodiment, single stranded nucleic acid probes have been modified to contain internally labels located on the probe. The probe labels are of different stability or susceptible to differential degradation depending on whether the target nucleic acid sequence is hybridized to the probe.

Notably, the present invention comprises assay systems whereby the label on the bound probe is stabilized relative to the unbound probe. This stabilization may be aided by intercalation. DNA intercalating compounds, including acridinium esters, are particularly, but not exclusively, suited for use in this inventive assay system. DNA intercalators are known to bind non-covalently to duplex DNA and are characteristically flat molecules which may insert between base pairs of the double helix of DNA. We have evidence which indicates that acridinium esters prefer to insert in regions rich in adenine and thymidine base pairs.

It should be understood that while the present invention hereinafter will be described with particular reference to acridinium ester labelled probes, the present invention contemplates the use of equivalent labels and assay formats which are susceptible to differential degradation or change in stability when the conjugate to which the label is a component is bound. As will be explained in more detail herein, other suitable label compounds include substances which are destabilized by the amines present on nucleic acids particularly those in the vicinity of the label on unhybridized probes. Moreover, label substances which can interact with hydrophobic environments, for example by intercalating between base pairs, also may be used.

This invention has application generally to any system involving the selective substantial degradation of the label when comparing its bound and unbound conjugated forms. Furthermore, because of the relative simplicity of the assay system, which does not involve any separation or washing steps, it is both faster and less expensive than conventional systems. The system can be more sensitive than conventional systems in those cases in which there are large differences in stability between the bound and unbound conjugated forms of the label because virtually all of the residual or unbound label conjugate is destroyed and, therefore, not detected. Finally, the system is very versatile and can be used either alone, or in combination with other separation methods to achieve very low background noise. The present invention is useful in probe diagnostics, including infectious and genetic and viral disease detection and cancer diagnosis.

### Brief Description of the Drawing

Figure 1 is a graphical representation of HPLC purification of an acridinium ester-labeled probe.

Figure 2 is a graphical representation of the results set forth in Example 2.

Figures 3-5 are graphical representations of the results set forth in Example 3.

Figure 6 is a graphical representation of the results set forth in Example 4.

Figure 7 is a graphical representation of the results set forth in Example 5.

Figure 8 is a graphical representation of the results set forth in Example 6.

### Best Mode and Detailed Description of the Preferred Embodiments

The following definitions shall be used in this description:
1. acridinium ester: derivative of acridine possessing a quaternary nitrogen center and derivatized at the 9 position to yield a labile phenyl ester moiety, specifically, 4-(2-succinimidyloxycarbonyl ethyl) phenyl-10-methylacridinium 9-carboxylate fluorosulfonate: 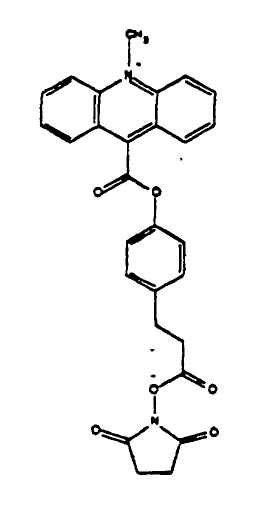
2. acridinium esters: moieties of the following general type: 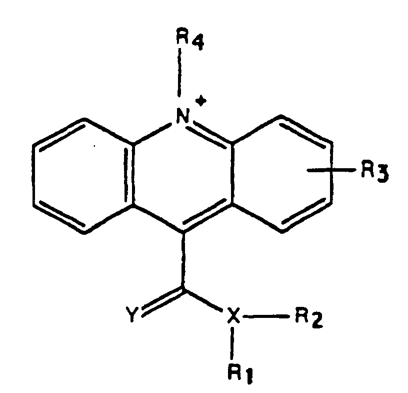
   - R₁=: ALKYL, ALKENYL, ARYL, SUBSTITUTED ALKYL. SUBSTITUTED ALKENYL, SUBSTITUTED ARYL, ALKOXY, ARYLOXY, OR IS ABSENT WHEN X=HALOGEN.
   - R₂ =: H, ALKYL, ALKENYL, ARYL, SUBSTITUTED ALKYL, SUBSTITUTED ALKENYL, SUBSTITUTED ARYL, ALKOXY, ARYLOXY, IF AND ONLY IF X=N.
   - R₃=: H, AMINO, HYDROXY, THIOL, HALOGEN, NITRO, AMINO, AMIDO, ACETYL, ALKYL, ALKENYL, ARYL, SUBSTITUTED ACETYL SUBSTITUTED, ALKYL, SUBSTITUTED ALKENYL, SUBSTITUTED ARYL, ALKOXY, ARYLOXY.
   - R₄=: ALKYL, ALKENYL, ARYL, SUBSTITUTED ALKYL, SUBSTITUTED ALKENYL, SUBSTITUTED ARYL
   - X=: O, N, S, HALOGEN, SUBSTITUTED PHOSPHOROUS, SUBSTITUTED SULFUR, SUBSTITUTED BORON, OR SUBSTITUTED ARSENIC.
   - Y=: O, S, OR NH.
   - R₁: AND/OR R₂ AND/OR R₃ AND/OR R₄ HAS A REACTIVE SITE WHICH ALLOWS CHEMICAL CONJUGATION.
3. analyte - any substance capable of undergoing a binding reaction with one or more specific binding partners, including, without limitation, antigens and antibodies thereto, haptens and antibodies thereto; hormones, drugs, metabolites, vitamins, coenzymes and their binding partners, including receptors; polynucleotides, oligonucleotides, and hybrids of polynucleotides or oligonucleotides and antibodies and binding substances thereto; polynucleotides or oligonucleotides and hybridizable polynucleotides or oligonucleotides thereto; metals and chelating agents thereto.
4. binding partner - any molecule or substance capable of undergoing a specific binding reaction with an analyte.
5. duplex: double stranded complex formed upon the annealing of two complementary, single stranded nucleic acid molecules.
6. hybridization: the formation of stable duplexes between 2 complementary single stranded DNA or RNA molecules or between a DNA and complementary RNA molecule. Duplex formation is specific for complementary base pairs, thereby reproducing the genetic code of the specific gene hybridized.
7. bound: condition in which a binding interaction has been formed between a molecule and its specific binding partner.
8. stable: resistant to chemical or biochemical degradation, reaction, decomposition, displacement or modification.
9. stability: the resistance of a substance to chemical or biochemical degredation, reaction, decomposition, displacement or modification.

It is known that in solution acridinium esters exist in equilibrium with their corresponding bases. At high pH, base formation is favored; the quaternary nitrogen species reforms at low pH. It also is known that chemiluminescence reaction can be affected by adding base, specifically an aqueous solution of sodium hydroxide containing hydrogen peroxide. The chemiluminescence involves attack by hydroperoxide ions on the acridinium species, which results in the formation of electronically excited N-methylacridone. See generally, Weeks et al. Acridinium Esters as High-Specific Activity Labels in Immunoassay, Clin. Chem. 2918, 1474-1479 (1983). The reaction is diagrammed below.

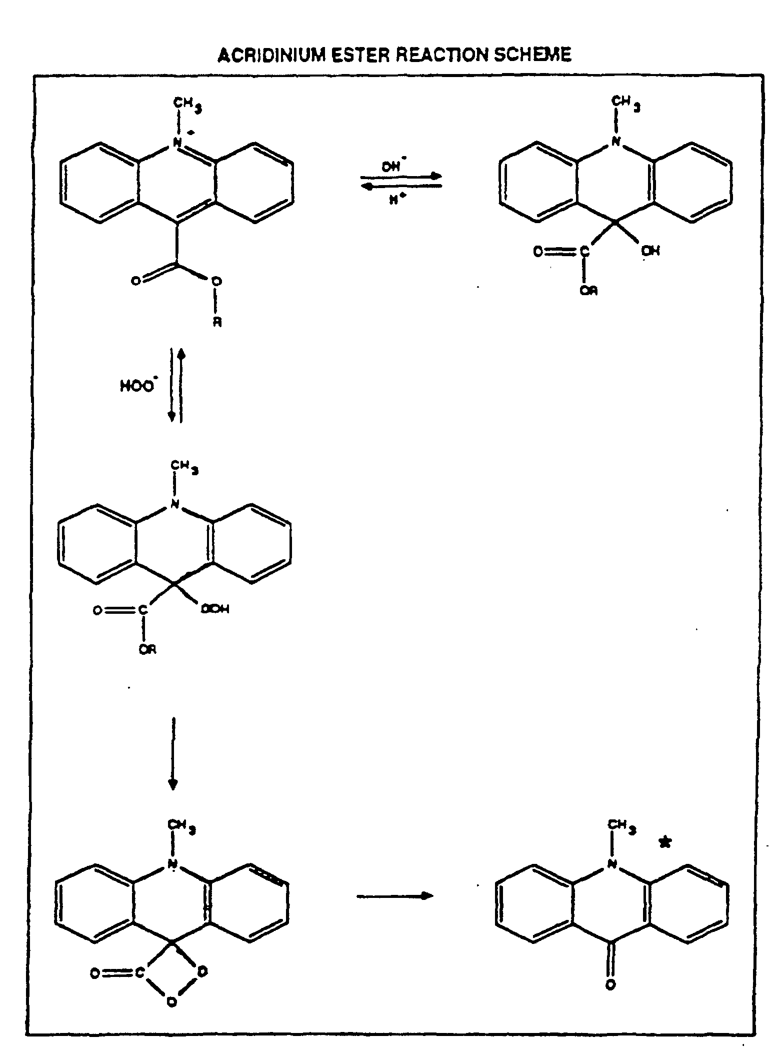

The subject invention can be carried out as follows. First, select binding partners comprising a binding substance and one or more binding partners for the assay to be performed. These pairs may be antigens and antibodies thereto; haptens and antibodies thereto; hormones, drugs, metabolites,vitamins, coenzymes and their binding partners, including receptors; polynucleotides, oligonucleotides, and hybrids of polynucleotides or oligonucleotides and antibodies and binding substances thereto; polynucleotides or oligonucleotides, and hybridizable polynucleotides or oligonucleotides thereto; metals and chelating agents therefor.

Second, select the assay format to be used. These may be selected from formats comprising direct binding assays, competition assays, sequential saturation methods, and sandwich assays.

Third, select a label for the assay to be performed. This may be a label which can be directly or indirectly detected by colorimetric, fluorimetric, chemiluminescent, or bioluminescent means. The label should additionally have the property that it can be chemically or biochemically degraded so as to modify its ability to be detected, said degradation being possible under conditions which do not adversely effect the binding between the labeled binding partner and its binding substance and other binding partners and binding substances which may participate in the reaction. Preferred labels are ones which are affected in their ability to be detected after exposure to acids, bases, or selective oxidizing agents such as peroxidate, or enzymes.

Fourth, using chemical methods known in the art, attach the label to the binding substance at a site such that the label's sensitivity to chemical or biochemical degradation is modified upon interaction of the labeled binding partner with its specific binding substance(s). In some cases several different sites may be tested for label attachment and the site which gives the best differential degradation may be used.

Fifth, optimize the degradation conditions, be they chemical or biochemical, to give the best detection discrimination of the labeled binding partner in the presence and absence of its binding substance.

Finally, using the preselected assay format, test the ability of the assay system to detect quantitatively or qualitatively the analyte generally employing the steps of:
a. Incubate
b. Selectively degrade
c. Detect
   or ;
a. Simultaneously incubate and selectively degrade
b. Detect.

Employing this invention, oligonucleotide probes labelled with chemiluminescent acridinium esters are particularly useful for the detection of sequence specific polynucleotides through hybridization. Acridinium esters may be attached at a number of different internal sites on DNA probes and/or mixed nucleotide/ non-nucleotide polymers as described in WO 89/02439. This includes the ability to label the nucleotide bases, the phosphate backbone, and the sugar residues of oligonucleotides as well as the nonnucleotide monomeric units of mixed nucleotide/non-nucleotides polymers.

Such acridinium ester labeled probes can show significant differential chemical stability when the probes to which they are attached are free in solution as compared to when they are hybridized. This differential stability is dependent upon the position of the acridinium ester in the probe, the nucleotide residues in the vicinity of the acridinium ester label and the presence of other probe molecules which form stable hybrids with the target polynucleotide. A graphic representation is set forth below.

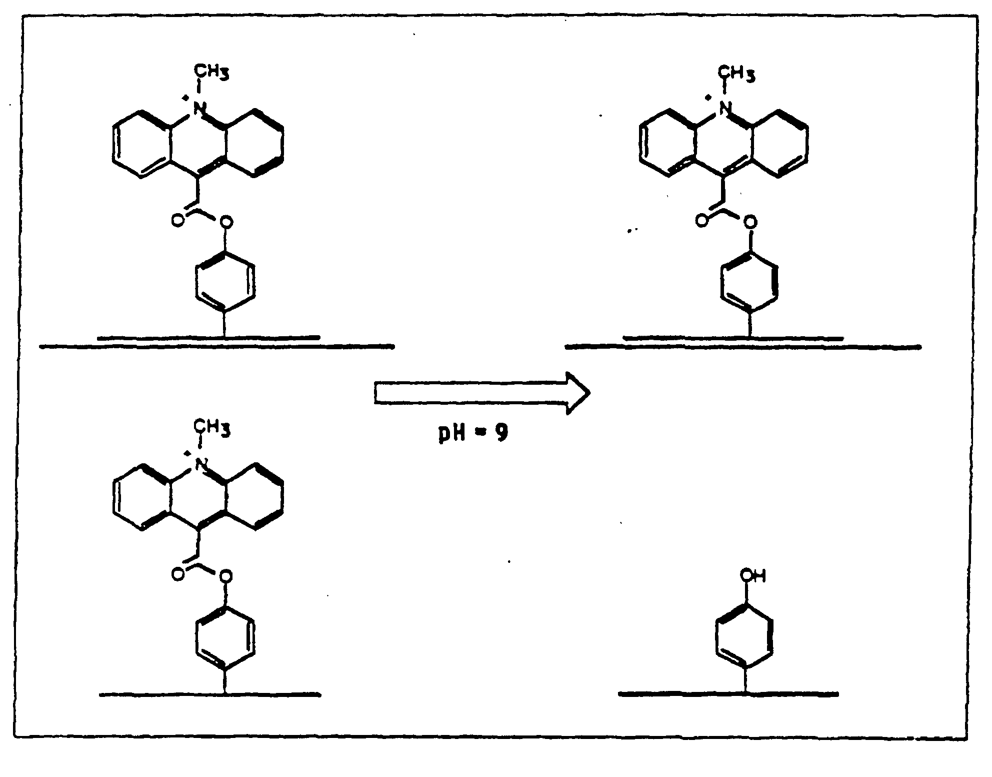

In the course of determining the factors which contribute to the differential stability of the acridinium ester on DNA probe molecules, we found that the free amines on the bases of unhybridized probes (both nucleotide and mixed nucleotide/non-nucleotide) destabilized the acridinium ester, especially in an alkaline environment. At the same time, if the acridinium ester is near the terminus of the probe, it can also be destabilized to alkali by amines contributed by target sequences once hybridization occurs. When the probe hybridizes with a sequence specific polynucleotide (binding substance) the probe amines participate in base pairing with complementary nucleotides and are restricted in their ability to destabilize the acridinium ester, especially in an alkaline environment. At the same time, it was found that the acridinium ester was further stabilized against degradation by intercalation into the hybrid duplex, particulary in regions of adenine/thymidine base pair density. We have found that a number of other insertion regions also give good differential hydrolysis, as explained in the following section.

There are several modes by which this invention may be applied to hybridization. These include without limitation:
1. Attaching the acridinium ester to the central region of the probe and near a region of adenine/thymidine base pairs. A preferred method is to attach an acridinium ester to a non-nucleotide monomeric unit as disclosed in WO 89/02439 which is attached as an insert to nucleotide monomeric units which are complementary to immediately adjacent nucleotides of the target polynucleotide sequence. Such placement serves to restrict the amines of the nucleotide bases on both sides of the acridinium ester and to provide a site for intercalation.
2. Attaching the acridinium ester at or near the site of a mismatch with a polynucleotide sequence that is not the desired target polynucleotide. In this manner discrimination between polynucleotide sequences differing by only one nucleotide can be achieved, since the area of the duplex around a mismatch site is sufficiently destabilized to render the acridinium ester (if it is attached at or near this site) susceptible to degradation.
3. Attaching acridinium ester to a probe region in order to monitor the local stability of a probe-target hybrid. In this manner, the acridinium ester can serve to closely monitor the local hybrid stability under conditions in which the entire hybrid duplex doesn't melt but under which the local hybrid region partially melts.

A preferred mode for the above three formats is to conduct the differential hydrolysis step at the same temperature as the hybridization step, typically at 50 to 70°C.

Another mode is to conduct a second differential hydrolysis step at room temperature. This allows pH's in the range of 10-11 to be used, yielding even larger differences in the rate of hydrolysis between hybridized and unhybridized acridinium ester-labeled probe.

### Experimental Methods and Materials

The following examples are offered by way of illustration. These examples are based on currently available data and the most likely explanations of the phenomenon. Other factors and methods may become evident with more research. Although the invention has been described in detail by way of illustration and examples using acridinium ester labels, certain changes and modifications may be practiced within the scope of the claims and other label systems also may be employed within the scope of the claims.

### Example 1.

### CONSTRUCTION OF ACRIDINIUM ESTER-LABELED PROBES

### A. Synthesis and Purification of Amine Linker-Arm Probes

Deoxyoligonucleotide probes were synthesized to contain an amine liner-arm (i.e., one which terminates in a primary amine for labeling with acridinium ester) located either at the 5'-terminus, at a specific preselected location along the polyphosphate chain, in the internal portion of the probe or attached to one of the nucleotide bases.

### (i)

To attach a 5'-amine linker-arm to a probe, the following compound was used:

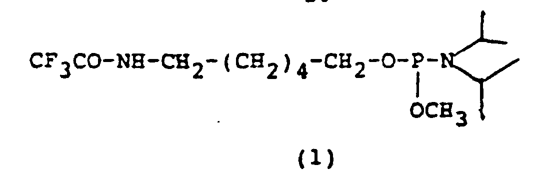

This compound will heretofore be referred to as terminal amine linker-arm reagent. This reagent was synthesized as follows: 6-amino hexanol was reacted with S-ethyltrifluorothioacetate in anhydrous ethylacetate. The reaction product was precipitated in petroleum ether, treated with a 10% pyridine in water mixture for 10 minutes to hydrolyze any 0-trifluoroacetyl which may have formed, and evaporated to dryness in the form of a gum. This compound was then phosphitylated according to standard protocols within the literature (see Nucleic Acids Research, 12 (11), 4539 (1984)) to yield the desired compound, namely, terminal amine linker-arm reagent (1).

Probes containing a 5'-amine linker-arm were synthesized as follows. Using an Applied Biosystems, Inc. Model 38OA DNA synthesizer, probes of desired nucleotide sequence were produced using standard phosphoramidite chemistry, building the probes from the 3' end to the 5' end. After the desired sequence was completed, the amine linker-arm was automatically coupled to the 5'-hydroxyl group of the probe using the terminal amine linker-arm reagent in the same way another phosphoramidite nucleoside would have been coupled. Using standard protocols, the probe was then cleaved from the solid support and deprotected using NH₄OH and purified by polyacrylamide gel electrophoresis followed by Sephadex G-25 chromatography.

The following probe was synthesized and purified using this procedure: 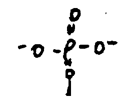 5'-NH2-(CH2)6-GCTCGTTGCGGGACTTAACCCAACAT-3' where NH₂-(CH₂)₆ represents the amine linker-arm.

### (ii)

To incorporate an amine linker-arm into the internal portion of a probe, internal amine linker-arm reagent, type 1 (4 atom spacer; see L1 in patent cited below), or type 2 (2 atom spacer; see L3 in patent cited below), or L7 (9 atom spacer) was used as described in WO 89/02439. Again, probes were synthesized using standard phosphoramidite chemistry and purified using polyacrylamide gel electrophoresis and Sephadex G25 chromatography.

The following probes were synthesized using this procedure:
1.) A 3Omer complementary to the 16S subunit of rRNA from E. coli, with an internal amine linker-arm, type 1, replacing an adenine residue at position 18 in the sequence: 
|Replaced with an internal amine linker arm, type 1|
5'-CCA CTG CTG CCT CCC GTA GGA GTC TGG GCC-3'.
2.) A 33mer complementary to the 16S subunit of rRNA from Chlamydia trachomatis, with an internal amine linker-arm, type 1, replacing an adenine residue at position 21 in the sequence, 
Replaced with an internal amine linker arm, type 1
5'-CGT TAC TCG GAT GCC CAA ATA TCG CCA CAT TCG-3',
 or inserted between residues 21 and 22,

An internal amine linker-arm, type 1 or type 2, inserted here
5'-CGT TAC TCG GAT GCC CAA ATA TCG CCA CAT TCG-3'.

Nucleotide bases containing amine linker-arm were incorporated into a probe as follows. Template and primer oligonucleotides with the following sequences were synthesized: Template 3' - GCA ATG AGC CTA CGG GTT TAT AGC GG - 5' Primer 5' - CGT TAC TCG GAT GCC CAA AT - 3' (The primer and extended primer sequences are complementary to the 16S subunit of C. trachomatis.)

Primer extension using the Klenow fragment was performed as described in Maniatis (Molecular Cloning, A Laboratory Manual, T. Maniatis, 1982, Cold Spring Harbor Laboratories, Pubs.) with the exception that the BSA was omitted from the 10X nick translation buffer, to incorporate the amine linker-arm modified base amino (12) dUTP (Calbiochem, California). The sequence of the resulting oligomer is, therefore:

CGT TAC TCG GAT GCC CAA ATA (amino-12-U)CG CC.

Purification of the primer extended complex was achieved using a NENSORB-20 cartridge (DuPont) following the procedure recommended by the manufacturer. (The primer extension reaction was diluted by the addition of 900 µl of NENSORB reagent A prior to loading on the column. The purified oligomers were eluted with 50% methanol which was then removed in a speed-vac.)

### B. Labeling of Amine Linker-Arm Probe with Acridinium Ester and Subsequent Purification

A 25mM stock solution of acridinium ester was prepared in distilled DMSO. The desired amount of probe (see a listing of the different probes labeled in section A above) was evaporated to dryness in a 1.5 ml conical polypropylene tube. The following cocktail was constructed by adding the following ingredients in the order listed:
3µl H₂O
1µl 1M HEPES (pH 8.0)
4µl DMSO (distilled)
2µl 25mM acridinium ester in DMSO (distilled)
The mixture was vortexed, spun in a microcentrifuge for 2 seconds (to bring the contents to the bottom of the tube), and incubated at 37°C for 20 minutes. The following components were then added to the reaction cocktail in the order listed:
3.0µl 25mM acridinium ester in DMSO (distilled)
1.5µl H₂O
0.5µl 1M HEPES (pH 8.0)
The cocktail again was vortexed, spun, and incubated an additional 20 minutes at 37°C. The unreacted label was quenched using a 5-fold excess of lysine by adding 5µl of 0.125M lysine in 0.1M HEPES (pH 8.0), 50% DMSO, and incubated 5 minutes at room temperature.

The acridinium ester-labeled oligomer was then purified using the following method. To the 20µl quenched reaction mixture 30µl 3M NaOAc (pH 5.0), 245µl H₂O and 5µl glycogen was added as a carrier (the glycogen was pre-treated to remove any nuclease activity). The sample was vortexed briefly and 640µl of absolute EtOH added. The sample was vortexed briefly and incubated on ice 5-10 minutes, then centrifuged 5 minutes at 15,000 rpm in a microcentrifuge. The supernatant was carefully removed and the pellet was redissolved in 20µl of 0.1M NaOAc (pH 5.0), 0.1% SDS. The samples were then purified by high performance liquid chromatography (HPLC) as described below.

### (i)

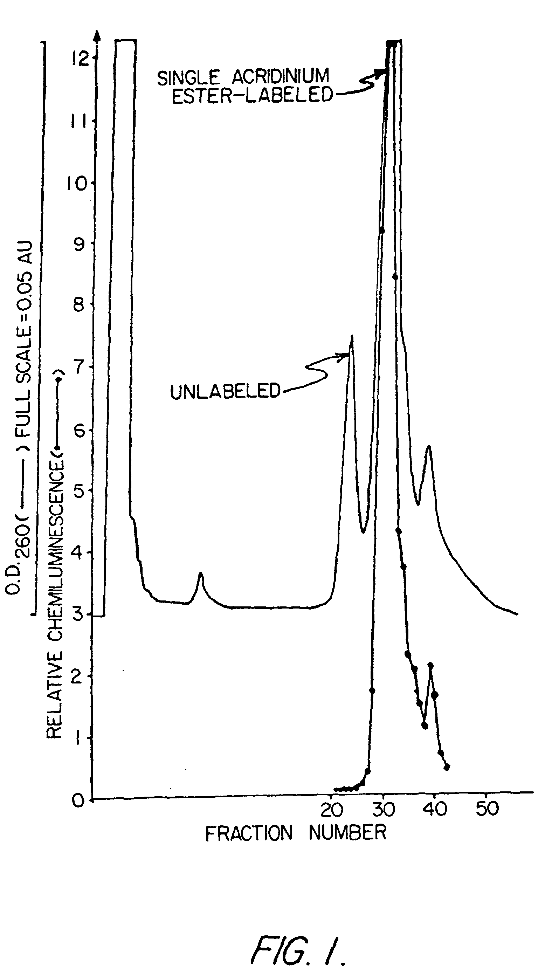

The AE-labeled probes containing the 5' and internal amine linker-arms were purified as follows: the 20µl redissolved pellet was injected onto a a Nucleogen-DEAE 60-7 ion-exchange HPLC column mounted in an IBM® 9533 HPLC system. All buffers were made with HPLC grade water, acetonitrile (CH₃CN) and sodium acetate (NaOAc) from Fisher Scientific, and reagent grade glacial acetic acid (HOAc) and LiCl. All buffers were filtered through 0.45µm pore size Nylon-66 filters before use. The sample was eluted as described in Figure 1 of the drawing (in this case the probe was the 5'-amine linker-arm containing 26mer described in Section A above). Immediately after the run, 5µl of 10% SDS was added to each tube followed by vortexing of each tube (this was done to ensure that the acridinium ester-labeled probe did not stick to the walls of the tube). A 0.5µl aliquot was removed from fractions 21-42 and added to 200µl water in a 12x75mm tube (a separate pipet tip was used for each aliquot to avoid a carryover problem). The chemiluminescence of each aliquot was then determined in a Berthold Clinilumat using the following automatic injection and read sequence: injection of 200µl of 0.25N HNO₃, 0.1% H₂O₂; a 1 second delay; a 200µl injection of 1N NaOH; read chemiluminescent output for 10 seconds.

Fractions 29-33 were then EtOH precipitated as follows: Add to each fraction 5µl glycogen, vortex, add 1 ml EtOH, vortex, incubate 5-10 minutes on ice, and centrifuge 5 minutes at 15,000 rpm in a microcentrifuge. Each supernatant was carefully removed, the pellet redissolved in 20µl 0.1M NaOAc, pH 5, 0.1% SDS, and the fractions pooled.

In this manner, highly pure acridinium ester-labelled probes were obtained. The specific activity of such probes was typically 5-10x10⁷ chemiluminescent light counts (Berthold Clinilumat) per picomole of oligomer.

### (ii)

The AE-labeled probe containing the amine linker-arm modified base (amino-12-U) was purified generally as described above, with the following exceptions: A Vydac C4 reverse-phase column was used; buffer A was 0.1M triethyammonium acetate (Applied Biosystems, Inc., Foster City, California) and buffer B was CH₃CN; the labeled probe was eluted as a hybrid using a linear gradient for 10-15% solvent B in 25 minutes at a flow rate of 1 ml/min. The main chemiluminescent peak was then identified and worked up as described above.

The preceding discussion generally describes how to make and label probes with acridinium ester. In the specific example herein, probes were end and internal labeled, as well as labeled in a nucleotide base.

### Example 2.

### STABILITY AT pH 6 OF HYBRIDIZED VERSUS UNHYBRIDIZED PROBE INTERNALLY LABELED WITH ACRIDINIUM ESTER

An internally labeled 33mer probe specific for Chlamydia trachomatis was prepared as previously described (adenine replacement, type 1 linker-arm). The probe was hybridized with its target rRNA (in this case Chlamydia trachomatis) according to the following procedure:

### Hybridization mixture

2µl AE-probe (0.5 pmol)
0.3µl 4% (wt:vol) SDS
5.8µl 1M PB, pH 5
4.4µl C. trachomatis rRNA (2µg), or 4.4µl H₂O for control.

The hybridization and control mixtures were incubated 40 minutes at 60°C, at which point each mixture was analyzed for percent hybridization using hydroxyapatite (HAP) as follows. A 0.1µl aliquot of the hybrid or control mixture was added to 200µl of 0.14M PB, pH 6.8, containing 2% HAP. Each resulting mixture was vortexed 5 seconds, incubated 5 minutes at 60°C, vortexed 20 seconds, then centrifuged 30 seconds in a microcentrifuge at 15,000 rpm. The supernatant was removed and saved, 200µl of 0.14M PB, pH 6.8, was added to the HAP pellet, the mixture was vortexed 10 seconds, then centrifuged 30 seconds in a microcentrifuge at 15,000 rpm. The supernatant was removed and saved and the pellet was resuspended in 200µl 0.14M PB, pH 6.8. A 50µl aliquot of the resuspended HAP and each of the 2 supernatants were analyzed for chemiluminescence as described below, and the percent hybridized probe, i.e., the percent chemiluminescent signal associated with the HAP, was calculated.

The stability of hybridized probe versus unhybridized probe (i.e., control) was tested at pH 6 according to the following procedure:

### Stability test mixture

12.3µl hybrid or control from above
50µl 1M PB, pH 6
2.5µl 4% SDS
65µl H₂O

These mixtures were vortexed briefly, and 5µl aliquots were removed immediately (tₒ) and analyzed for chemiluminescence as described below. The remainder of the mixtures were incubated at 60°C, and 5µl aliquots were removed at various time points (see below) and analyzed immediately for chemiluminescence.

The chemiluminescence of each sample was measured by adding a sample aliquot to 200µl H₂O in a 12x75mm tube and measuring chemiluminescence in a Clinilumat (automatic injection of 200µl 0.25N HNO₃, 0.1% H₂O₂ followed after a 1 second delay, by auto-injection of 200µl 2M potassium PB, pH 13.2, and reading of chemiluminescence for 10 seconds).

### RESULTS:

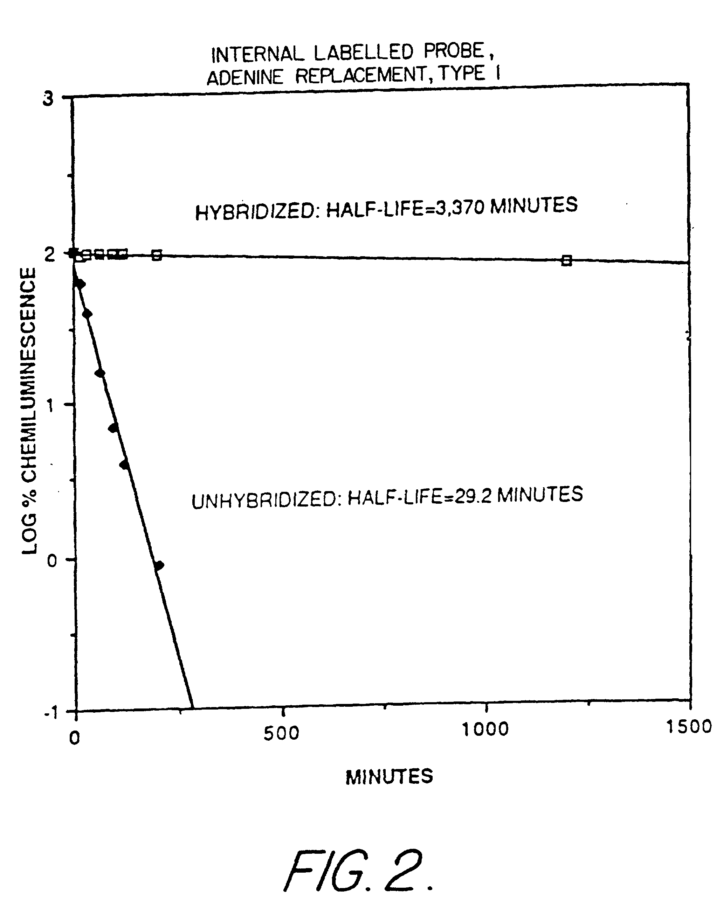

1. Percent Hybridization (HAP analysis)
   Hybrid - 96%
   Control - 1.3% (non-specific binding)
2. Stability time course
   See Fig. 2 of the drawing.

These results demonstrate that the hybridized probe is well protected against breakdown and subsequent loss of chemiluminescense (half-life for loss of chemiluminescence equal to 3370 minutes), whereas unhybridized probe is very susceptible to breakdown and loss of chemiluminescence (half-life is equal to 29.2 minutes; therefore, discrimination between hybridized and unhybridized is equal to 115-fold). These data demonstrate the ability of the homogeneous assay herein described to locate target DNA sequences by imparting differential stability between hybridized and unhybridized probe and measuring same.

### Example 3.

### STABILITY AT pH 9 OF HYBRIDIZED VERSUS UNHYBRIDIZED PROBE INTERNALLY LABELED WITH ACRIDINIUM ESTER

Internally labeled probe (all 3 internally labeled 33mer probes described in Example 1 were tested) was hybridized with its target rRNA (in this case Chlamydia trachomatis) and analyzed for percent hybridization as described in Example 2.

The stability of hybridized versus unhybridized probe (i.e., control) was tested at pH 9 according to the following protocol:

### Stability test mixture

5µl hybrid or control from above
45µl 0.2M sodium borate, pH 9
The mixtures were then incubated, sampled and analyzed for chemiluminescence as described in Example 2.

### RESULTS:

1. Percent Hybridization (HAP analysis)
   a. Adenine replacement, type 1 linker-arm
      Hybrid - 95%
      Control - 0.5% (non-specific binding)
   b. Insertion, type 1 linker-arm
      Hybrid - 98%
      Control - 0.3%
   c. Insertion, type 2 linker-arm
      Hybrid - 98%
      Control - 0.2%
2. Stability time course

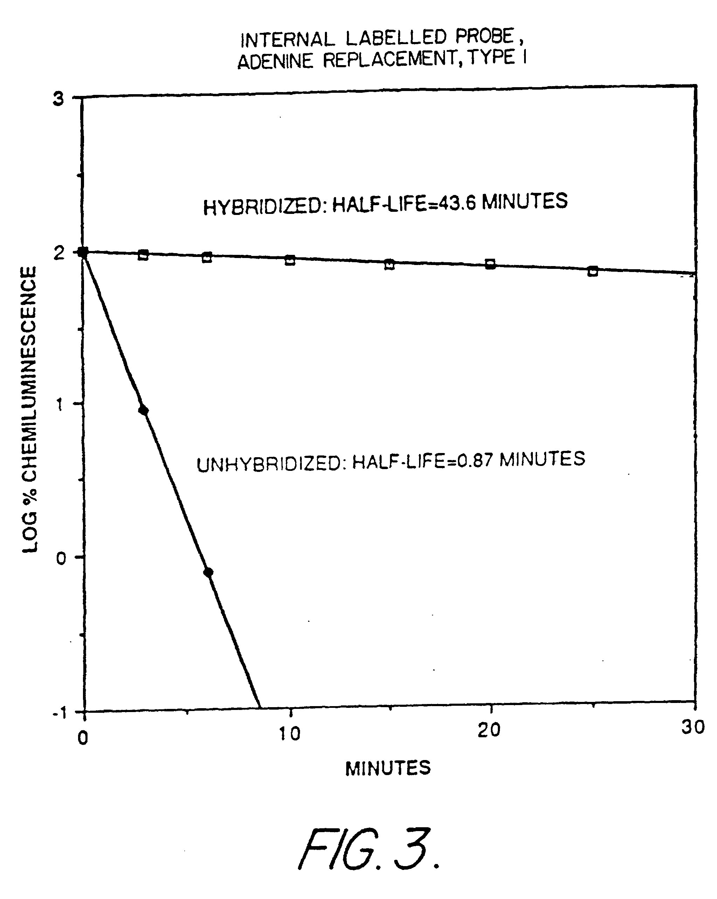

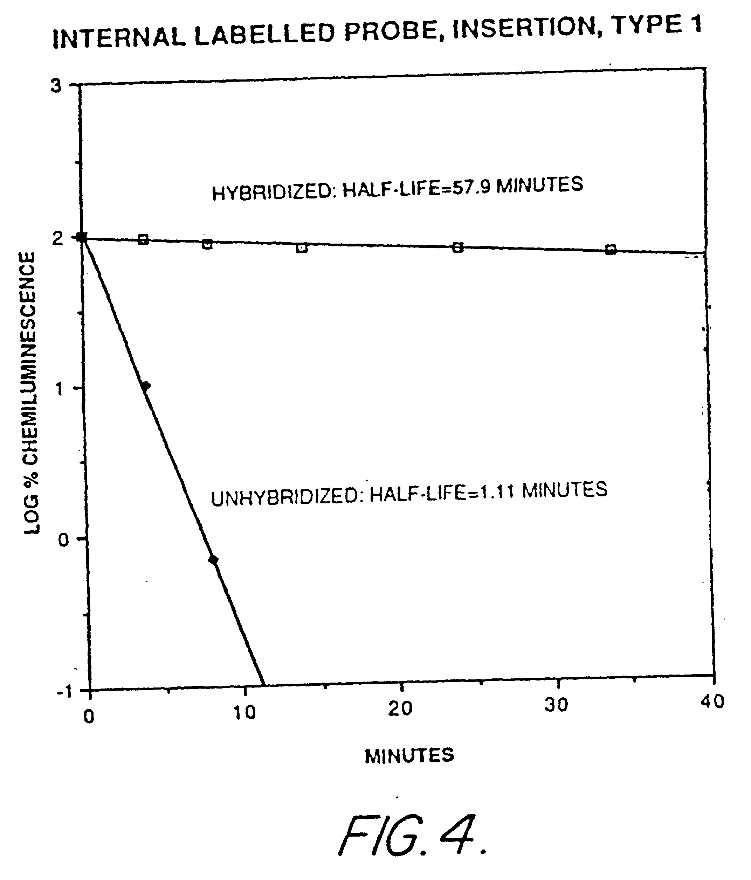

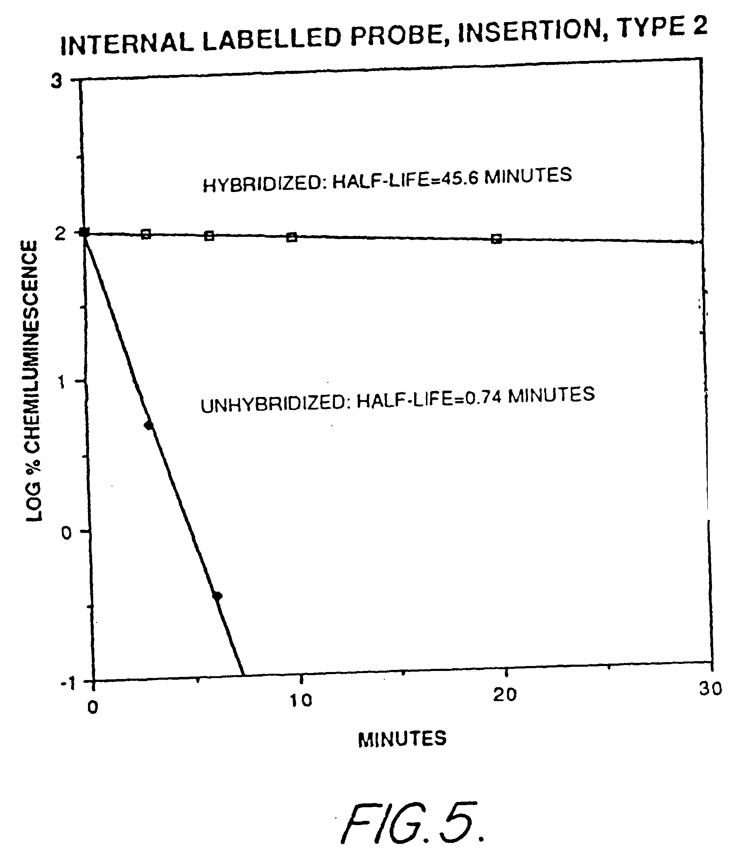

See Figs. 3-5 of the drawing. Fig. 3 is a graph for adenine replacement, type 1 linker-arm; Fig. 4 is a graph of insertion, type 1 linker-arm; Fig. 5 is a graph of insertion, type 2 linker-arm.

As in Example 2, hybridized probe is protected from degradation whereas unhybridized probe is not. This is true for all three types of internally labeled probes. In this example, sodium borate at elevated pH is shown to accelerate this process (as compared with example 2) while still retaining the differential degredation characteristics between hybridized and unhybridized probe.

### Example 4.

### STABILITY AT pH 6 OF PROBE END LABELED WITH ACRIDINIUM ESTER AS HYBRID WITH ADJACENT PROBE VERSUS NON-HYBRID

This example involves use of a probe of sequence 5'-CCG GAC CGC TGG CAA CAA AGG ATA AGG GTT GC-3' (prepared using standard phosphoramidite chemistry) which hybridizes to E. coli rRNA immediately adjacent to the 5' end of the AE-labeled probe used in this example (see below), thereby leading to the "capping off" of all the amines in the target rRNA in the vicinity of the acridinium ester label.

Probe end-labeled with acridinium ester (preparation described in Example 1) (hereinafter referred to as AE-probe) and the adjacent probe described above were hybridized according to the following procedure:

| Hybridization mixture | Control mixture |
|---|---|
| 1µl AE-probe (.25 pmol) | 1µl AE-probe (.25 pmol) |
| 2µl E. coli rRNA (2ug) | 5.4µl H₂O |
| 4.3µl adjacent probe (12 pmol) | 5.8µl 1M PB, pH 5 |
| 0.3µl 4% SDS | 0.3µl 4% SDS |
| 7.5µl 1M PB, pH 5 | |

The hybridization and control mixtures were incubated 40 minutes at 60°C, and then analyzed for percent hybridization using hydroxyapatite as described in Example 2.

The stability of the hybridized probe with adjacent probe versus unhybridized probe (i.e., control) was tested at pH 6 exactly as described in Example 2.

### RESULTS:

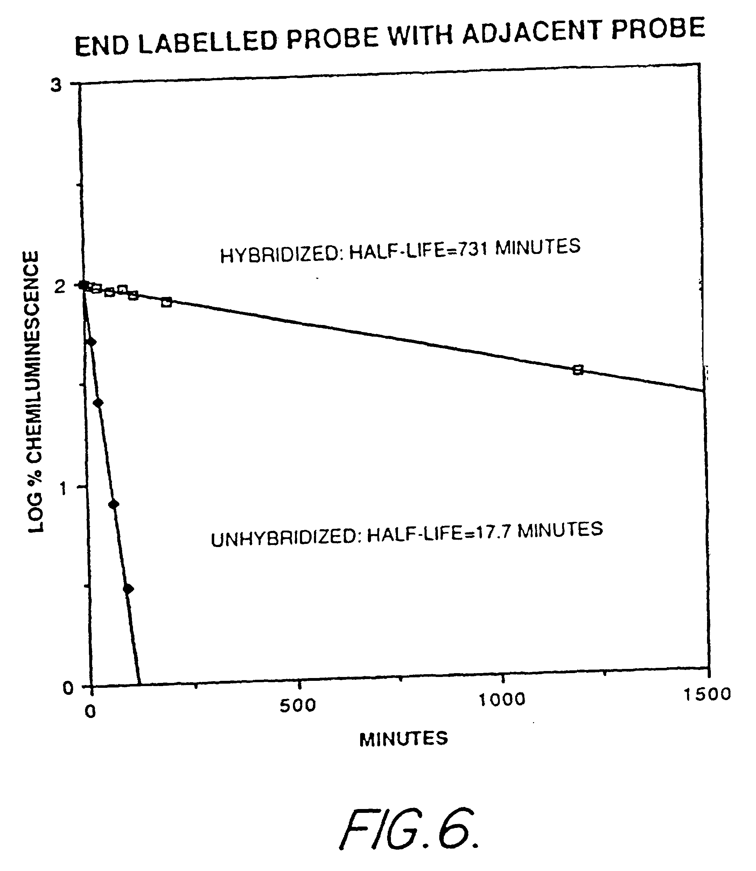

1. Percent hybridization (HAP analysis)
   Hybrid - 93.5%
   Control - 2.7% (non-specific binding)
2. Stability time course.
   See Fig. 6 of the drawing.

As in Example 2, hybridized AE-probe, in this case with an adjacent probe also hybridized, was protected from degradation whereas unhybridized probe was not. The protection was as good as in Example 2, because it is dependent on two hybridations instead of one.

### Example 5.

### STABILITY AT pH 9 OF PROBE END LABELED WITH ACRIDINIUM ESTER AS HYBRID WITH "ADJACENT" PROBE VERSUS NON-HYBRID.

Hybridization was carried out exactly as described in Example 4. Stability of hybridized probe with adjacent probe versus unhybridized probe (i.e., control) was tested at pH 9 exactly as described in Example 2, except the composition of the stability test mixture, which was as follows:
5µl hybrid or control
50µl 0.2M sodium borate, pH 9.0

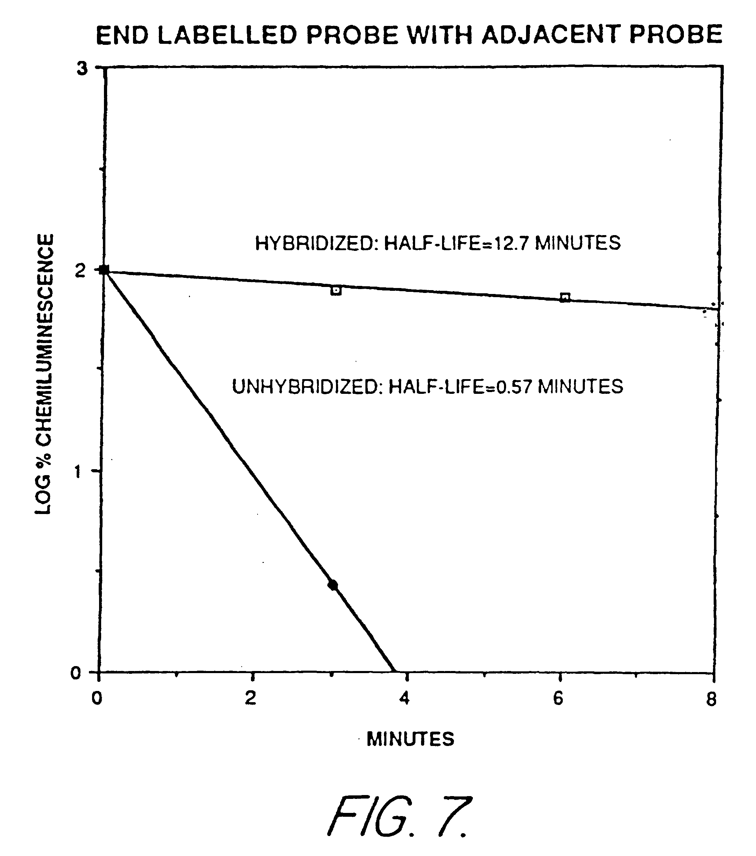

Hybridized AE-probe with an adjacent probe (also hybridized) again was protected from degradation whereas unhybridized probe was not. As in Example 3, sodium borate at elevated pH accelerated the process while still retaining the differential degradation characteristics between hybridized and unhybridized probe. See Fig. 7 for a graphical representation of these results.

### Example 6.

### STABILITY AT pH 6 OF PROBE END LABELED WITH ACRIDINIUM ESTER AS HYBRID VERSUS NON-HYBRID.

Probe was hybridized to its target rRNA (in this case E. coli) according to the following procedure:

| Hybridization mixture | Control mixture |
|---|---|
| 1µl AE-probe (.25 pmol) | 1µl AE-probe (.25 pmol) |
| 2µl E. coli rRNA (2µg) | 5.4µl H₂O |
| 5.8µl 1M PB, pH 5 | 5.8µl 1M PB, pH 5 |
| 0.3µl 4% SDS | 0.3µl 4% SDS |
| 3.4µl H₂O | |

The hybridization and control mixtures were incubated 40 minutes at 60°C, and then analyzed for percent hybridzation using hydroxyapatite as described in Example 2.

The stability of the hybridized probe versus unhybridized probe (i.e., control) was tested at pH 6 exactly as described in Example 2.

### RESULTS:

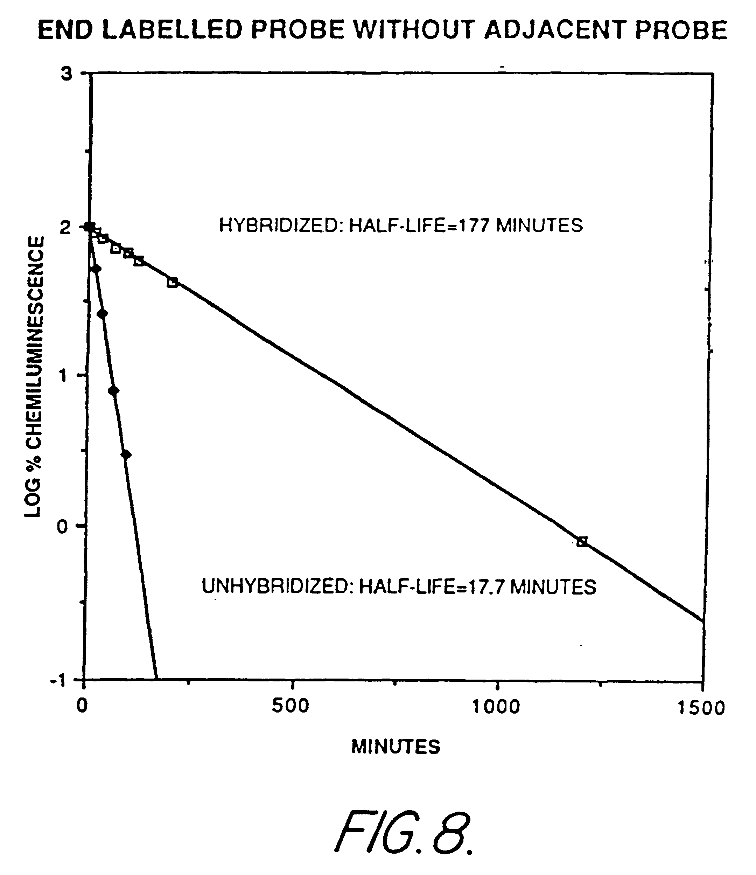

1. Percent hybridization
   Hybrid - 94%
   Control - 2.7% (non-specific binding)
2. Stability time course
   See Fig. 8 of the drawing.

Unhybridized probe again was preferentially degraded as compared to hybridized probe, although the differential in degradation is not as great as in previous examples. This is because only a portion of the amines were "capped off" in the vicinity of the acridinium ester label. Indeed, this additionally demonstrates the ability to discriminate between hybridized end-labeled probe in the presence and absence of hybridized adjacent probe.

### Example 7.

### STABILITY AT pH 7.6 OF HYBRIDIZED VERSUS UNHYBRIDIZED PROBE LABELED ON A NUCLEOTIDE BASE WITH ACRIDINIUM ESTER

The probe labeled on a nucleotide base (amino-12-U) with acridinium ester described in Example 1, was hybridized with its target rRNA (in this case C. trachomatis) according to the following procedure:

### Hybridization Mixture

0.1M lithium succinate, pH 5.4
10% lithium lauryl sulfate
2 µg (1.3pmol) C. trachomatis rRNA or water for control
0.1 pmol AE- probe
Total volume - 30 µl

The hybridization and control mixtures were incubated 5 minutes at 80°C, followed by 60 minutes at 60°C. The resulting solutions were each diluted to 300 µl with 0.1M lithium succinate, pH 5.4, 10% lithium lauryl sulfate, and analyzed for percent hybridization using hydroxyapatite as described in Example 2.

The stability of hybridized versus unhybridized probe (i.e., control) was tested at pH 7.6 with several identically prepared samples according to the following protocol:

### Stability test mixture

15 µl hybrid or control from above
100 µl 0.2% sodium tetraborate, pH 7.6, 5% Triton X-100

These mixtures were then incubated at 60°C, and samples were removed at various time points and chemiluminescence was determined using a Gen-Probe Leader™ I Luminometer employing the automatic injection of 200 µl of 0.1M H₂O₂, a 1-second delay, the automatic injection of 200 µl of IN NaOH, and reading of chemiluminescence for 5 seconds. From these data the rates of hydrolysis were determined by regression analysis.

### Results:

1. Percent Hybridization (HAP Analysis)
Hybrid - 53.8%
Control - 0.5%
2. Stability

| Half-life of ester hydrolysis (min) | | Ratio of half-lives |
|---|---|---|
| Hybrid | Control | (Hybrid/Control) |
| 13.6 | 1.3 | 10.5 |

As in preceding examples, these data demonstrate that hybridized probe is protected against degradation whereas unhybridized probe is not, in this case with the label attached to a base of the probe. This demonstrates the principle that different types of sites are acceptable for AE attachment for use in the homogeneous protection assay described herein.

### Example 8.

### STABILITY AT pH 7.6 OF HYBRIDIZED VERSUS UNHYBRIDIZED PROBES INTERNALLY LABELED WITH ACRIDINIUM ESTER AT A VARIETY OF SEQUENCE DIMER SITES USING BOTH rRNA AND DNA TARGETS

Probes containing the internal linker-arm, type L7, inserted at a variety of sequence dimer sites (see below) were synthesized, labeled with AE, and purified as described in Example 1. The sequence and linker-arm locations of these probes are as follows:

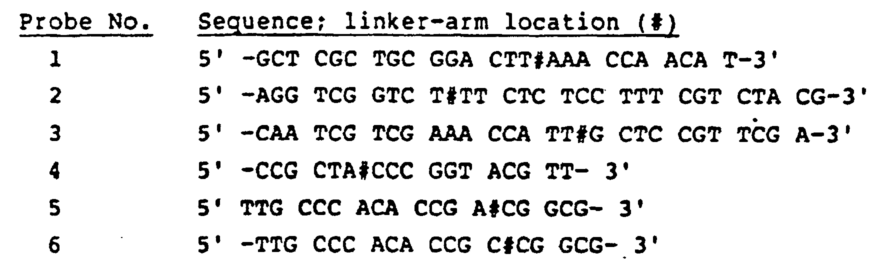

These probes were hybridized as described in Example 7 with the exception that the 80°C incubation step was omitted, and the amounts and types of target nucleic acids used were as follows:

| Probe # | Target Nucleic Acid |
|---|---|
| 1 | 1 µg of E. coli rRNA |
| 2 & 3 | 1 µg of C. trachomatis rRNA |
| 4 | 1 µg of N. gonorrhoeae rRNA |
| 5 & 6 | 1.2 pmol of the exact synthetic DNA complement |

The stability of hybridized versus unhybridized probe was tested at pH 7.6 as described in Example 7.

### RESULTS:

### Stability

| Ratio of half-lives | | | |
|---|---|---|---|
| Half-life of ester hydrolysis (min) | | | Hybrid/Control |
| Probe# | Hybrid | Control | |
| 1 | 41.2 | 0.94 | 43.7 |
| 2 | 24.2 | 0.71 | 34.5 |
| 3 | 40.7 | 0.96 | 42.4 |
| 4 | 15.7 | 1.2 | 13.1 |
| 5 | 11.1 | 1.0 | 11.1 |
| 6 | 22.6 | 0.74 | 30.5 |

These data demonstrate that the linker-arm (and therefore the AE) can be inserted at a wide variety of sequence dimer sites and still be substantially protected against ester hydrolysis in the hybridized form. Furthermore this example demonstrates that DNA targets provide good protection of hybridized AE-probes when employed with these homogeneous assay formats. This example also demonstrates that the long (9 atom) linker-arm used here yields differential hydrolysis ratios essentially equivalent to the other linker-arms cited earlier in this patent establishing that a wide variety of linker-arm lengths can be used in the HPA format described herein.

### Example 9.

### STABILITY AT pH 7.6 OF INTERNALLY LABELED AE-PROBE HYBRIDIZED WITH PERFECTLY MATCHED TARGET VERSUS TARGET WITH A SINGLE MISMATCH

A 24mer probe complementary to the 16S subunit of rRNA from E. coli, with an internal amine linker-arm, type L7, inserted between residues 6 and 7 was synthesized. The probe was then labeled with acridinium ester and purified and described in Example 1. The sequence is as follows (# = linker-arm position):

5' -CAA GCT# TGC CAG TAT CAG ATG CAG- 3'

This probe has a single, T-G mismatch at position 6 (numbered from the 5' end of the probe strand) with the 16S subunit of C. diversius.

The probe was hybridized with its target rRNA (in this case either E. coli or C. diversius) according to the procedure described in Example 8. The stability of the resulting hybridized probes, as well as a sample of unhybridized probe was tested at pH = 7.6 as described in Example 7.

### Stability

| Ratio of half-lives | | | |
|---|---|---|---|
| Half-life of ester hydrolysis (min) | | | Hybrid/Control |
| Target | Hybrid | Control | |
| E. coli | 18.6 | 0.8 | 23.3 |
| C. diversius | 1.1* | 0.8 | 1.4 |

| | | | |
|---|---|---|---|
| * The low half-life of ester hydrolysis was not due to low hybridization extent, as HAP analysis (as described in Example 2) revealed 73% hybridization. | | | |

These data show that in the case of the hybrid with the perfectly matched target the AE-probe is protected from ester hydrolysis (as described in previous examples), whereas in the hybrid with the target containing a single base mismatch the AE-probe is poorly protected. This gives rise to a 17-fold difference in hydrolysis rate of the AE-probe between the perfect target and a single site mismatch target. Therefore, the method described herein is readily able to discriminate between nucleic acid targets containing single base differences.

### Example 10.

### STABILITY AT ROOM TEMPERATURE UNDER VARIOUS CONDITIONS OF HYBRIDIZED VERSUS UNHYBRIDIZED PROBE INTERNALLY LABELED WITH ACRIDINIUM ESTER

Internally labeled probe (insertion, type 1; see Example 1) was hybridized with 1 µg of C. trachomatis rRNA as described in Example 8. The stability of the hybridized versus unhybridized probe was measured at room temperature under a variety of reagent and pH conditions (see "Results") according to the procedure described in Example 7.

### RESULTS:

| | | HALF LIVES (MINUTES) | | | HALF LIFE |
|---|---|---|---|---|---|
| BUFFER | pH | TEMP | HYBRID | CONTROL | RATIO |
| 0.2M borate 5% Triton | 7.6 | 60°C | 34.13 | 0.89 | 38.08 |
| 0.2M borate 5% Triton | 7.6 | 40°C | 272 | 7.5 | 36.26 |
| 0.2M borate 5% Triton | 7.6 | Room Temp. | 2307.7 | 61 | 37.82 |
| 0.2M borate 5% Triton | 9.0 | Room Temp. | 468.75 | 7.7 | 60.87 |
| 0.2M borate 5% Triton | 10.0 | Room Temp. | 98.36 | 1.3 | 73.78 |
| 0.2M borate 5% Triton | 11.0 | Room Temp. | 5.6 | 0.79 | 7.08 |
| 50mM phytic acid, 0.05% SDS | 10.0 | Room Temp. | 145.48(fast)* 398.55(slow)* | 2.18 | 66.73(fast)* 182.82(slow)* |
| 50mM phytic acid, 0.05% SDS | 11.0 | Room | 175.44 | 1.31 | 133.92 |

| | | | | | |
|---|---|---|---|---|---|
| *In the phytic acid system, the hydrolysis kinetics were biphasic. "Fast" refers to the early, fast phase of hydrolysis, and "slow" refers to the later, slow phase of hydrolysis. | | | | | |

These date demonstrate that there are a wide variety of conditions under which the homogeneous protection assay described herein will function, making it adaptable to a wide range of assay conditions. Furthermore, systems other than borate are acceptable, for example, the phytic acid system at room temperature where very high half-life ratios are achieved.

### Example 11.

### DETECTION OF A DILUTION SERIES OF CHLAMYDIA rRNA IN BUFFER USING PROBE INTERNALLY LABELED WITH ACRIDINIUM ESTER

Internally labeled probe (as in Example 2) was hybridized to decreasing amounts of its target rRNA (in this case Chlamydia trachomatis) according to the following procedure:

### Hybridization mixture

0.5µl probe (12.5 fmol)
1µl RNA (10⁻², 10⁻³, 10⁻⁴ or 10⁻⁵µg)
2µl 20% SDS
1.7µl H₂O
4.8µl 1M PB, pH 6.8

The control mixture was the same as hybridization mixture except that it contained water instead of rRNA, and the reagent blank mixture was the same as the control mixture except that it contained water instead of probe. The mixtures were incubated 20 minutes at 60'C.

After hybridization, 90µl of 0.2M borate, pH 9, was added to each sample, followed by incubation at 60°C for 14 minutes. Each sample was then read for chemiluminescence as described in Example 2, except that injection 1 was 0.1% H₂O₂ only, injection 2 was pH 13.8 instead of 13.2, and the read time was 7 seconds instead of 10 seconds.

### RESULTS:

Reagent blank - 116 rlu
Control - 124 rlu

| | Minus control |
|---|---|
| 10⁻⁵µg rRNA - 126 rlu | 2 rlu |
| 10⁻⁴µg rRNA - 210 rlu | 86 rlu |
| 10⁻³µg rRNA - 1100 rlu | 976 rlu |
| 10⁻²µg rRNA - 7809 rlu | 7685 rlu |

Reagent blank and control represent average of triplicates; all others represent average of duplicates.

These results demonstrate that the invention described herein was able to detect a linear dilution series of target rRNA to a limit of sensitivity of about 10⁻⁴µg in a pure buffer system.

### Example 12.

### DETECTION OF A DILUTION SERIES OF CHLAMYDIA rRNA IN CLINICAL MEDIA USING PROBE INTERNALLY LABELED WITH ACRIDINIUM ESTER

Internally labeled probe (as in Example 2) was hybridized in clinical specimen (throat swab) to decreasing amounts of its target rRNA (in this case Chlamydia trachomatis) according to the following procedure:

### Hybridization mixture

50µl throat swab
6µl 4.8M PB, pH 4.7
2µl rRNA (3x10⁻⁴, 3x10⁻³, 3x10⁻² or 3x10⁻¹µg)
2µl probe (1 pmol)

Control mixture was the same as hybridization mixture except that it contained water instead of rRNA, and the reagent blank mixture was the same as control mixture except that it contained water instead of probe. The mixtures were incubated 60 minutes at 60°C.

After hybridization, one third of each mixture (20µl) was added to 50µl 0.2M borate, final pH = 9 (after addition of hybridization mixtures), followed by incubation at 60°C for 40 minutes. Each sample was then read for chemiluminescence as described in Example 11.

### RESULTS:

| | | |
|---|---|---|
| Reagent blank | 163 rlu | |
| Control | 6560 rlu | |
| | | |

| | Minus control | |
|---|---|---|
| 10⁻³µg rRNA | 8535 rlu | 1975 |
| 10⁻²µg rRNA | 27306 rlu | 20746 |
| 10⁻¹µg rRNA | 258194 rlu | 251634 |

Data represent average of duplicate values.

These data demonstrate that the invention described herein was able to detect a linear dilution series of target rRNA to a limit of sensitivity of about 10⁻³µg in a system containing clinical media.

### Example 13.

### DETECTION OF A DILUTION SERIES OF BACTERIAL rRNA IN URINE USING PROBE INTERNALLY LABELED WITH ACRIDINIUM ESTER

An internally labeled 30mer probe specific for E. coli was prepared as previously described (adenine replacement, type 1 linker-arm). The probe was hybridized in urine to decreasing amounts of its target rRNA (in this case E. coli) according to the following procedure:

### Hybridization mixture

79.6µl urine
0.4µl 0.25M EDTA, 0.25M EGTA
10µl 4.8M PB, pH 6.8
5µl 20% SDS
5µl probe (.125 pmol)
1µl RNA (10⁻³, 10⁻², or 10⁻¹µg)

Control mixture was the same as hybridization mixture except that it contained water instead of rRNA, and the reagent blank mixture was the same as control mixture except that it contained water instead of probe. The mixtures were incubated 30 minutes at 60°C.

After hybridization, 300µl 0.2M borate, pH 9, was added to each sample, followed by incubation at 60°C for 16 minutes. Each sample was then read for chemiluminescence as described in Example 11.

### RESULTS:

| | | |
|---|---|---|
| Reagent blank | 6845 rlu | |
| Control | 9250 rlu | |
| | | |

| | Minus Control | |
|---|---|---|
| 10⁻³µg rRNA | 9358 rlu | 8 rlu |
| 10⁻²µg rRNA | 14055 rlu | 4805 rlu |
| 10⁻¹µg rRNA | 61800 rlu | 52550 rlu |

Results represent the average of duplicate values.

These data demonstrate that the invention described herein was able to detect a linear dilution series of target rRNA to a limit of sensitivity of approximately 5x10⁻³µg RNA in a system containing urine.

### Example 14.

### SELECTIVE DEGRADATION USED IN COMBINATION WITH A SEPARATION STEP FOR DETECTION OF A DILUTION SERIES OF CHLAMYDIA rRNA IN CLINICAL MEDIA USING PROBE INTERNALLY LABELED WITH ACRIDINIUM ESTER

Internally labeled probe (as in Example 2) was hybridized in clinical specimen (throat swab) as described in Example 8, including control and blank mixtures. After hybridization, one-third of each mixture was removed and subjected to selective degradation exactly as described in Example B, while one-third was simply removed and allowed to stand at room temperature (i.e., no selective degradation). Both sets of samples, namely, with and without selective degradation, were then subjected to separation of hybridized from unhybridized probe using HAP as described in Example 2 with the following exceptions;
a. 1 ml of HAP solution was used (instead of 200µl),
b. 1 ml of wash solution was used (instead of 200µl),
c. 3 washes were performed (instead of 1),
d. The final HAP pellets were resuspended in 100µl of wash solution, the entirety of which was measured for chemiluminescence (as described in Example 2).

### RESULTS:

| SELECTIVE DEGRADATION | | | | |
|---|---|---|---|---|
| Minus | S:B | Plus | S:B | |
| Control (no rRNA) | 18 | -- | 4.7 | -- |
| 10⁻³ µg rRNA | 27 | 1.5 | 10 | 2.1 |
| 10⁻² µg rRNA | 117 | 6.5 | 70 | 15 |
| 10⁻¹ µg rRNA | 933 | 52 | 591 | 126 |
| 0.33 µg rRNA | 2756 | 153 | 1755 | 373 |

Results represent the average of duplicative values with reagent blank already substracted. S:B = signal to background ratio, i.e., chemiluminescence at a particular rRNA concentration divided by chemiluminescence of control.

These data demonstrate that the addition of selective degradation before separation results in lower backgrounds leading to higher signal to background ratios, and therefore improved sensitivity.

### Example 15.

### IMPROVED STRINGENCY USING DIFFERENTIAL HYDROLYSIS

The following probe was constructed to contain an internal linker-arm, type L7 (position of insertion indicated by # below), labeled with acridinium ester, and purified as described in Example 1:

ATT CCG CAC A#TG TCA AAA CCA G

This probe is exactly complementary to the 16S subunit of Neisseria gonorrhoeae. However, it is also closely related to N. meningitidis, and a cross-reaction is typically observed under the hybridization stringency conditions cited in Examples 2 and 8. This probe was hybridized with 0.5 µg N. meningitidis as described in Example 8, (except in a 200 µl format), then either incubated for 10 minutes at 60°C in 1 ml of 0.2M sodium tetraborate, pH 7.6, 5% Triton X-100 to effectuate differential hydrolysis (+ D.H.), or not subjected to these differential hydrolysis conditions (-D.H.). The resulting hybrids were then separated on magnetic microspheres and measured for chemiluminescence as described below.

Add 1 ml of 0.4M PB, pH 6.0, containing 150 µg of magnetic amine microspheres (BioMag M4100, Advanced Magnetics, Inc., Cambridge, Mass.). Vortex 10 seconds. Incubate at 60°C for 5 minutes. Vortex 10 seconds. Magnetically separate the spheres from the solution using the Pace-Mate™ magnetic separation rack (Gen-Probe, Inc., San Diego, CA). Discard liquid. Wash the spheres by adding 1 ml of 0.3M PB, pH 6.0, vortexing for 10 seconds, magnetically separating and discarding the liquid. Repeat for a total of three washes. Elute the hybrid by adding 300 µl of 0.2M PB, pH 6.0, 50% formamide, vortexing 10 seconds, incubating at 60°C for 5 minutes, vortexing for 10 seconds, and magnetically separating the spheres from the solution. Transfer the solution to a Clinilumat tube and measure chemiluminescence as described in Example 7.

### RESULTS:

| Condition | Chemiluminescence* |
|---|---|
| - D.H. | 178,034 |
| + D.H. | 748 |

| | |
|---|---|
| *Hybrid signal minus control (i.e., no rRNA) signal, given in relative light units (rlu). Signal from the exact target (i.e., N. gonorrhoeae) in this format is typically 7-10 X 10⁶ rlu. | |

In conclusion, application of the differential hydrolysis technique described herein as an additional stringency discrimination step greatly reduces signal from undesired, cross-reacting sequences. In this example cross-reaction of a N. gonorrhoeae probe with N. meningitidis was lowered greater than 200-fold. The differential hydrolysis step increases sensitivity to unstable hybrids, which are in equilibrium with unhybridized probe, by constantly reducing the chemiluminescence (via hydrolysis) of the probe when it is in the unhybridized form, thus lowering the chemiluminescence due to cross reaction.

### Example 16.

### DETECTION OF A CHIMERIC TARGET SEQUENCE ASSOCIATED WITH CHRONIC MYELOGENOUS LEUKEMIA USING A PROBE INTERNALLY LABELED WITH ACRIDINIUM ESTER

A 24mer probe (sequence given below) with an internal linker-arm, type L7, inserted as indicated was synthesized, labeled with AE and purified as described in Example 1. This probe is complementary to the chimeric mRNA transcript (common break) associated with chronic myelogenous leukemia (CML) and will be called the bcr/abl probe. This chimeric mRNA is a product of the chimeric gene formed by the translocation of a region of the abl gene on chromosome 9 into a region of chromosome 22 containing the bcr gene.

Synthetic DNA 60mer targets representing the breakpoint junction region of the bcr/abl chimeric target, as well as the normal bcr and abl targets in that same region, were synthesized as described in Example 1 (sequences given below). Also, an mRNA target was produced by transcription of a pGEM clone containing a 450 nucleotide segment of the chimeric bcr/abl gene centered around the breakpoint. An mRNA transcript of the same 450 nucleotide segment in the probe sense was also produced as a negative control.

BCR/ABL PROBE SEQUENCE

5'-CCGCTGAAGGGCTTTT*GAACTCTGC-3'

### SYNTHETIC TARGET SEQUENCE

BCR/ABL TARGET

5'ACTCAGCCACTGGATTTAAGCAGAGTTCAAAGCCCTTCAGCGGCCAGTAGCATCTGACT-3'

ABL TARGET

5'-ACTCAGCCACTGGATTTAAGCAGAGTTCAATCTGTACTGCACCCTGGAGGTGGATTCCT-3'

The asterisk inserted above denotes a linker-arm insertion site for acridinium ester attachment and the underline indicates abl sequences.

The bcr/abl probe was hybridized with approximately 1 pmol of each of the targets listed above as described in Example B, and the stability of these hybrids and the unhybridized probe was tested at pH 7.6 as described in Example 7.

### RESULTS:

| half-life (min) | ratio* | |
|---|---|---|
| Targets: bcr/abl mRNA | 27.7 | 39.6 |
| bcr/abl DNA | 15.0 | 21.4 |
| Controls: probe sense mRNA | 0.7 | 1 |
| abl DNA | 0.7 | 1 |
| bcr DNA | 0.7 | 1 |
| Unhybridized probe | 0.7 | -- |

| | | |
|---|---|---|
| *Target or control half-life divided by unhybridized probe half-life. | | |

These data demonstrate that an AE-labeled probe designated to span the breakpoint junction region of the chimeric bcr/abl mRNA transcript associated with CML was able to discriminate between chimeric target and normal sequences (as well as unhybridized probe) using the HPA technology described herein. This is a demonstration that the method of the invention can be used to specifically detect chimeric targets (typically associated with genetic disorders) in the presence of the normal, non-chimeric component nucleic acids.

This example further demonstrates that targets other than rRNA-in this case both mRNA and DNA - afford protection against AE hydrolysis when hybridized to AE-labeled probe.

## Claims

1. A single-stranded polymeric sequence comprising at least three nucleotide monomers or at least two nucleotide monomers and at least one non-nucleotide monomer, and a label, wherein said label is internally located, provided that the stability of said label present internally in a nucleic acid hybrid comprising said single-stranded polymeric sequence and a complementary nucleic acid differs from the stability of said label present in said single-stranded polymeric sequence not hybridized to nucleic acid.

2. The single-stranded polymeric sequence of claim 1, wherein said label is internally located at least five nucleotide monomers from the 3' and 5' ends of said single-stranded polymeric sequence.

3. The single-stranded polymeric sequence of claim 1, wherein said label is internally located at least six nucleotide monomers from the 3' and 5' ends of said single-stranded polymeric sequence.

4. The single-stranded polymeric sequence of any one of claims 1-3, wherein said single-stranded polymeric sequence comprises a non-nucleotide monomer and said label is located on said non-nucleotide monomer.

5. The single-stranded polymeric sequence present in any one claims 1-4, wherein said label may be detected by either colorimetric, fluorimetric, chemiluminescent or bioluminescent means.

6. The single-stranded polymeric sequence of claim 5, wherein said label may be detected by either fluorimetric or chemiluminescent means.

7. The single-stranded polymeric sequence of claim 6, wherein said label may be detected by chemiluminescence induced through a chemical reaction on an acridinium species forming an electronically excited acridone species.

8. The single-stranded polymeric sequence of claim 6, wherein said label comprises an acridinium ester.

9. The single-stranded polymeric sequence of claim 8, wherein said label has the following chemical structure: 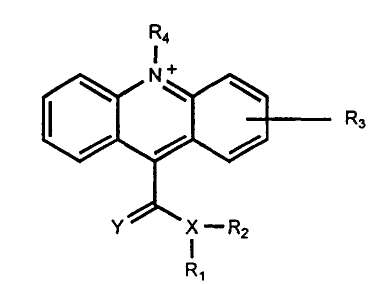
wherein X is selected from the group consisting of O, N, S, halogen, substituted phosphorous, substituted sulfur, substituted boron, and substituted arsenic;
Y is selected from the group consisting of O, S, and NH;
R₁ is either alkyl, alkenyl, aryl, substituted alkyl, substituted alkenyl, substituted aryl, alkoxy, or aryloxy, or is absent when X is a halogen;
R₂ is either H, alkyl, alkenyl, aryl, substituted alkyl, substituted alkenyl, substituted aryl, alkoxy or aryloxy, only if X is N, otherwise R₂ is absent;
R₃ is selected from the group consisting of H, amino, hydroxy, thiol, halogen, nitro, amido, acetyl, alkyl, alkenyl, aryl, substituted acetyl, substituted alkyl, substituted alkenyl, substituted aryl, alkoxy or aryloxy;
R₄ is selected from the group consisting of alkyl, alkenyl, aryl, substituted alkyl, substituted alkenyl and substituted aryl; and
wherein said label is joined to said single-stranded polymeric sequence through a sterically tolerant site connecting to either R₁, R₂, R₃, or R₄.

10. A composition comprising the single-stranded polymeric sequence of any one of claims 1-9; and
a polynucleotide base sequence,
wherein a region of said polynucleotide sequence is able to form a polymeric sequence:polynucleotide hybrid with a region of said single-stranded polymeric sequence, and wherein the stability of said label present in said polymeric sequence:pclynucleotide hybrid differs from the stability of said label in the absence said polymeric sequence:polynucleotide hybrid.

11. The composition of claim 10, wherein said single-strand polymeric sequence is hybridized to said polynucleotide base sequence to produce said polymeric sequence:polynucleotide hybrid.

12. The composition of claims 10 or 11, wherein said single-stranded polymeric sequence and said polynucleotide base sequence can form a hybrid which is not perfectly complementary wherein said label is positioned on said single-stranded polymeric sequence at or near a nucleoside base not perfectly complementary with said polynucleotide base sequence, such that said label is more susceptible to a change in stability than if said nucleoside base was opposite a portion of said polynucleotide base sequence perfectly complementary to said polynucleotide base sequence.

13. The composition of claim 12, wherein said label is positioned on said single-stranded polymeric sequence in a location adjacent to a mismatch present in said polymeric sequence:polynucleotide hybrid.

14. A composition comprising,
a first single-stranded polymeric sequence comprising a label at or near its 3' or 5' end,
a second single-stranded polymeric sequence, and
a polynucleotide base sequence comprising a target region,
wherein said first single-stranded polymeric sequence and said second single-stranded polymeric sequence are both able to hybridize to said target region at adjacent regions such that the stability of said label in the presence of a hybridization complex comprising said first single-stranded polymeric sequence, said second single-stranded polymeric sequence, and said target region, is greater than the stability of said label when present on said first single-stranded polymeric sequence which is single-stranded or which is hybridized to said target region in absence of said second single-stranded polymeric sequence.

15. A method of detecting the presence or amount of a nucleic acid analyte target sequence in a sample comprising the steps of:
a) contacting said sample with the single-stranded polymeric sequence of any one of claims 1-9, wherein said single-stranded polymeric sequence can hybridize to said target sequence to form a probe:target complex comprising said single-stranded polymeric sequence and said target sequence if said analyte is present in said sample, wherein the stability of said label in said probe:target complex differs from the stability of said label not present in said probe:target complex;
b) treating said sample so as to cause a difference in the ability of said label to be detected when not present in said probe:target complex compared to when said label is present in said probe:target complex;
c) detecting the presence of signal from said label present in said probe:target complex, or from said label not present in said probe:target complex, as a measure of the presence of or amount of said analyte.

16. The method of claims 15, wherein said single-stranded polymeric sequence not present in said probe:target complex is an unhybridized nucleic acid probe, and said step (c) detects the presence of signal from said label present in said probe:target complex as a measure of the presence of or amount of said analyte.

17. The method of claim 15, wherein said label not present in said probe:target complex may be present in a probe:non-target complex comprising said single-stranded polymeric sequence and a non-target nucleic acid, and said step (c) detects the presence of signal from said label present in said probe:target complex as a measure of the presence of or amount of said analyte.

18. The method of claim 17, wherein said non-target nucleic acid differs from said target sequence by at least one nucleotide.

19. The method of any one of claims 15-18, further comprising separation of said single-stranded polymeric sequence in said probe:target complex from said single-stranded polymeric sequence not in said probe:target complex prior to said step (c).

20. A method for detecting the presence or amount of a nucleic acid analyte target sequence in a sample comprising the steps of:
a) contacting said sample with the first and second single-stranded polymeric sequence of claim 14, wherein said first and second single-stranded polymeric sequence can hybridize to said target sequence to form a probe:target complex comprising said first, said second, and said target sequence if said analyte is present in said sample, wherein the stability of said label in said probe:target complex differs from the stability of said label not present in said probe:target complex;
b) treating said label under conditions wherein the stability of said label differs in said probe:target complex from the stability of said label not present in said probe:target complex so as to cause a difference in the ability of said label to be detected when not present in said probe:target complex compared to when said label is present in said probe:target complex;
c) detecting the presence of signal from said label present in said probe:target complex, or from said label not present in said probe:target complex, as a measure of the presence of or amount of said analyte.

21. The method of claim 20, wherein said first single-stranded polymeric sequence not present in said probe:target complex is an unhybridized nucleic acid probe, and said step (c) detects the presence of signal from said label present in said probe:target complex as a measure of the presence of or amount of said analyte.

22. The single-strand polymeric sequence of any one of claims 1-9, or the composition of any one of claims 10-14, wherein stability is resistance to degradation.

## Patentansprüche

1. Einzelsträngige Polymersequenz, umfassend mindestens drei Nukleotid-Monomere oder mindestens zwei Nukleotid-Monomere und mindestens ein nicht-Nukleotid-Monomer, und eine Markierung, wobei diese Markierung intern lokalisiert ist, vorausgesetzt, dass die Stabilität dieser Markierung, die in einem Nukleinsäure-Hybrid, umfassend die einzelsträngige Polymersequenz und eine komplementäre Nukleinsäure, intern vorhanden ist, von der Stabilität dieser Markierung abweicht, die in der einzelsträngigen, nicht an Nukleinsäure hybridisierten Polymersequenz vorhanden ist.

2. Einzelsträngige Polymersequenz nach Anspruch 1, wobei die Markierung mindestens fünf Nukleotid-Monomere von den 3'- und 5'-Enden der einzelsträngigen Polymersequenz entfernt intern lokalisiert ist.

3. Einzelsträngige Polymersequenz nach Anspruch 1, wobei die Markierung mindestens sechs Nukleotid-Monomere von den 3'- und 5'-Enden der einzelsträngigen Polymersequenz entfernt intern lokalisiert ist.

4. Einzelsträngige Polymersequenz nach einem der Ansprüche 1 bis 3, wobei die einzelsträngige Polymersequenz ein nicht-Nukleotid-Monomer umfasst und die Markierung an dem nicht-Nukleotid-Monomer lokalisiert ist.

5. Einzelsträngige Polymersequenz nach einem der Ansprüche 1 bis 4, wobei die Markierung entweder durch kolorimetrische, fluorimetrische, chemilumineszierende oder biolumineszierende Mittel nachweisbar ist.

6. Einzelsträngige Polymersequenz nach Anspruch 5, wobei die Markierung entweder durch fluorimetrische oder chemilumineszierende Mittel nachweisbar ist.

7. Einzelsträngige Polymersequenz nach Anspruch 6, wobei die Markierung durch Chemilumineszenz nachweisbar ist, wie durch eine chemische Reaktion an einer Acridinium-Spezies, was eine elektronisch angeregte Acridon-Spezies bildet, induziert wird.

8. Einzelsträngige Polymersequenz nach Anspruch 6, wobei die Markierung einen Acridiniumester umfasst.

9. Einzelsträngige Polymersequenz nach Anspruch 8, wobei die Markierung die folgende chemische Struktur aufweist: 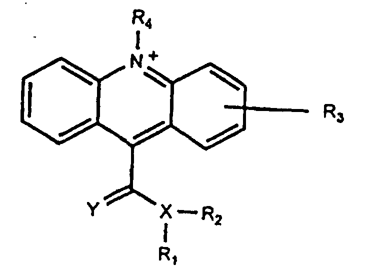
worin X gewählt ist aus der Gruppe, bestehend aus O, N, S, Halogen, substituiertem Phosphor; substituiertem Schwefel, substituiertem Bor und substituiertem Arsen;
Y gewählt ist aus der Gruppe, bestehend aus O, S und NH;
R₁ entweder Alkyl, Alkenyl, Aryl, substituiertes Alkyl, substituiertes Alkenyl, substituiertes Aryl, Alkoxy oder Aryloxy ist oder abwesend ist, wenn X ein Halogen ist;
R₂ entweder H, Alkyl, Alkenyl, Aryl, substituiertes Alkyl, substituiertes Alkenyl, substituiertes Aryl, Alkoxy oder Aryloxy ist, nur dann, wenn X N ist, und ansonsten R₂ abwesend ist;
R₃ gewählt ist aus der Gruppe, bestehend aus H, Amino, Hydroxy, Thiol, Halogen, Nitro, Amido, Acetyl, Alkyl, Alkenyl, Aryl, substituiertes Acetyl, substituiertes Alkyl, substituiertes Alkenyl, substituiertes Aryl, Alkoxy oder Aryloxy;
R₄ gewählt ist aus der Gruppe, bestehend aus Alkyl, Alkenyl, Aryl, sbustituiertem Alkyl, substituiertem Alkenyl und substituiertem Aryl; und
worin die Markierung an die einzelsträngige Polymersequenz durch eine sterisch tolerante Stelle geknüpft ist, die entweder zu R₁, R₂, R₃ oder R₄ verbindet.

10. Zusammensetzung, umfassend die einzelsträngige Polymersequenz nach einem der Ansprüche 1-9; und
eine Polynukleotid-Basensequenz,
worin eine Region dieser Polynukleotidsequenz zur Bildung eines Polymersequenz:Polynukleotid-Hybrids mit einer Region der einzelsträngigen Polymersequenz in der Lage ist und wobei die Stabilität der Markierung, die in diesem Polymersequenz:Polynukleotid-Hybrid vorhanden ist, von der Stabilität dieser Markierung in Abwesenheit des Polymersequenz:Polynukleotid-Hybrids abweicht.

11. Zusammensetzung nach Anspruch 10, wobei die einzelsträngige Polymersequenz an die Polynukleotid-Basensequenz zum Erhalt des Polymersequenz:Polynukleotid-Hybrids hybridisiert ist.

12. Zusammensetzung nach Ansprüchen 10 oder 11, wobei die einzelsträngige Polymersequenz und die Polynukleotid-Basensequenz einen Hybrid bilden können, der nicht perfekt komplementär ist, wobei die Markierung auf der einzelsträngigen Polymersequenz an oder nahe einer Nukleosid-Base positioniert ist, die nicht perfekt komplementär mit der Polynukleotid-Basensequenz ist, so dass diese Markierung anfälliger für eine Stabilitätsveränderung ist als dann, wenn die Nukleosid-Base einem Abschnitt der Polynukleotid-Basensequenz gegenüberläge, der perfekt komplementär zur Polynukleotid-Basensequenz ist.

13. Zusammensetzung nach Anspruch 12, wobei die Markierung an der einzelsträngigen Polymersequenz in einer Lokalisierung positioniert ist, die angrenzend an eine in dem Polymersequenz:Polynukleotid-Hybrid vorhandenen Fehlpaarung liegt.

14. Zusammensetzung, umfassend:
eine erste einzelsträngige Polymersequenz, umfassend eine Markierung an oder nahe ihrem 3'- oder 5'-Ende,
eine zweite einzelsträngige Polymersequenz, und
eine Polynukleotid-Basensequenz, umfassend eine Zielregion,
wobei die erste einzelsträngige Polymersequenz und die zweite einzelsträngige Polymersequenz beide zur Hybridisierung an die Zielregion in angrenzenden Regionen in der Lage sind, so dass die Stabilität der Markierung in Gegenwart eines Hybridisierungskomplexes, umfassend die erste einzelsträngige Polymersequenz, die zweite einzelsträngige Polymersequenz und die Zielregion, größer ist als die Stabilität der Markierung, wenn auf der ersten einzelsträngigen Polymersequenz vorhanden, welche einzelsträngig ist oder welche an die Zielregion in Abwesenheit der zweiten einzelsträngigen Polymersequenz hybridisiert hat.

15. Verfahren zum Nachweisen des Vorhandenseins oder der Menge einer Zielsequenz in einem Nukleinsäure-Analyt einer Probe, umfassend die folgenden Schritte:
a) Zusammenbringen der Probe mit der einzelsträngigen Polymersequenz nach einem der Ansprüche 1-9, wobei die einzelsträngige Polymersequenz an die Zielsequenz hybridisieren kann und dabei einen Sonde:Zielkomplex bildet, umfassend die einzelsträngige Polymersequenz und die Zielsequenz, sofern das Analyt in der Probe vorhanden ist, wobei die Stabilität der Markierung in dem Sonde:Zielkomplex von der Stabilität der Markierung abweicht, die nicht in dem Sonde:Zielkomplex vorhanden ist;
b) Behandeln der Probe, um eine Differenz in der Nachweisbarkeit der Markierung zu bewirken, wenn diese nicht im Sonde:Zielkomplex vorhanden ist, im Vergleich dazu, wenn die Markierung im Sonde:Zielkomplex vorhanden ist;
c) Nachweisen des Vorhandenseins eines Signals von der Markierung, die im Sonde:Zielkomplex vorhanden ist, oder von der Markierung, die nicht in dem Sonde:Zielkomplex vorhanden ist, als ein Maß des Vorhandenseins oder der Menge des Analyts.

16. Verfahren nach Anspruch 15, wobei die nicht im Sonde:Zielkomplex vorhandene einzelsträngige Polymersequenz eine unhybridisierte Nukleinsäure-Sonde ist, und der Schritt (c) das Vorhandensein eines Signals von der Markierung, die im Sonde:Zielkomplex vorhanden ist, als ein Maß des Vorhandenseins oder der Menge des Analyts nachweist.

17. Verfahren nach Anspruch 15, wobei die nicht im Sonde:Zielkomplex vorhandene Markierung in einem Sonde:nicht-Zielkomplex vorhanden sein kann, umfassend die einzelsträngige Polymersequenz und eine nicht-Zielnukleinsäure, und der Schritt (c) das Vorhandensein eines Signals von der im Sonde:Zielkomplex vorhandenen Markierung als ein Maß des Vorhandenseins oder der Menge des Analyts nachweist.

18. Verfahren nach Anspruch 17, wobei die nicht-Zielnukleinsäure von der Zielsequenz in mindestens einem Nukleotid abweicht.

19. Verfahren nach einem der Ansprüche 15-18, außerdem umfassend:
Trennen der einzelsträngigen Polymersequenz in dem Sonde:Zielkomplex von der einzelsträngigen Polymersequenz, die nicht in dem Sonde:Zielkomplex vorhanden ist, vor dem Schritt (c).

20. Verfahren zum Nachweisen des Vorhandenseins oder der Menge einer Zielsequenz in einem Nukleinsäure-Analyt einer Probe, umfassend die folgenden Schritte:
a) Zusammenbringen der Probe mit der ersten und zweiten einzelsträngigen Polymersequenz nach Anspruch 14, wobei die erste und zweite einzelsträngige Polymersequenz an die Zielsequenz zum Erhalt eines Sonde:Zielkomplex hybridisieren kann, umfassend die erste einzelsträngige Polymersequenz, die zweite einzelsträngige Polymersequenz und die Zielsequenz, sofern das Analyt in der Probe vorhanden ist, wobei die Stabilität der Markierung in dem Sonde:Zielkomplex von der Stabilität der nicht in dem Sonde:Zielkomplex vorhandenen Markierung abweicht;
b) Behandeln der Markierung unter Bedingungen, wobei die Stabilität der Markierung in dem Sonde:Zielkomplex von der Stabilität der nicht in dem Sonde:Zielkomplex vorhandenen Markierung abweicht, wodurch eine Differenz in der Nachweisbarkeit der Markierung bewirkt wird, wenn diese nicht in dem Sonde:Zielkomplex vorhanden, verglichen dazu, wenn die Markierung im Sonde:Zielkomplex vorhanden ist;
c) Nachweisen des Vorhandenseins eines Signals von der im Sonde:Zielkomplex vorhandenen Markierung, oder von dem nicht im Sonde:Zielkomplex vorhandenen Markierung, als ein Maß des Vorhandenseins oder der Menge des Analyts.

21. Verfahren nach Anspruch 20, wobei die erste einzelsträngige Polymersequenz, die nicht in dem Sonde:Zielkomplex vorhanden ist, eine unhybridisierte Nukleinsäure-Sonde ist, und der Schritt (c) das Vorhandensein eines Signals von der Markierung, die in dem Sonde:Zielkomplex vorhanden ist, als ein Maß des Vorhandenseins oder der Menge des Analyts nachweist.

22. Einzelsträngige Polymersequenz nach einem der Ansprüche 1-9, oder die Zusammensetzung nach einem der Ansprüche 10-14, wobei die Stabilität im Widerstand gegen einen Abbau besteht.

## Revendications

1. Séquence polymérique monocaténaire contenant au moins trois monomères nucléotidiques ou au moins deux monomères nucléotidiques et au moins un monomère non nucléotidique, et un marqueur, dans laquelle ledit marqueur est situé en position interne, à condition que la stabilité dudit marqueur présent en position interne dans un hybride d'acide nucléique comprenant ladite séquence polymérique monocaténaire et un acide nucléique complémentaire diffère de la stabilité dudit marqueur présent dans ladite séquence polymérique monocaténaire non hybridée à l'acide nucléique.

2. Séquence polymérique monocaténaire de la revendication 1, dans laquelle ledit marqueur est situé en position interne à au moins cinq monomères nucléotidiques des extrémités 3' et 5' de ladite séquence polymérique monocaténaire.

3. Séquence polymérique monocaténaire de la revendication 1, dans laquelle ledit marqueur est situé en position interne à au moins six monomères nucléotidiques des extrémités 3' et 5' de ladite séquence polymérique monocaténaire.

4. Séquence polymérique monocaténaire de l'une quelconque des revendications 1 à 3, ladite séquence polymérique monocaténaire comprenant un monomère non nucléotidique et ledit marqueur étant situé sur ledit monomère non nucléotidique.

5. Séquence polymérique monocaténaire présente dans l'une quelconque des revendications 1 à 4, dans laquelle ledit marqueur peut être détecté par des moyens colorimétriques, fluorimétriques, chimioluminescents ou bioluminescents.

6. Séquence polymérique monocaténaire de la revendication 5, dans laquelle ledit marqueur peut être détecté par des moyens fluorimétriques ou chimioluminescents.

7. Séquence polymérique monocaténaire de la revendication 6, dans laquelle ledit marqueur peut être détecté par une chimioluminescence induite par l'intermédiaire d'une réaction chimique sur une espèce acridinium en formant une espèce acridone électroniquement excitée.

8. Séquence polymérique monocaténaire de la revendication 6, dans laquelle ledit marqueur comprend un ester d'acridinium.

9. Séquence polymérique monocaténaire de la revendication 8, dans laquelle ledit marqueur a la structure chimique suivante : 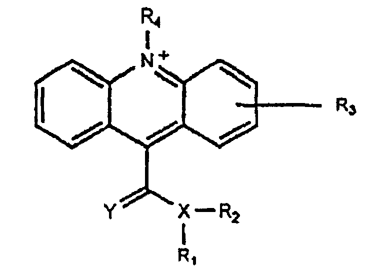
dans laquelle X est choisi dans l'ensemble consistant en O, N, S, halogène, phosphore substitué, soufre substitué, bore substitué, et arsenic substitué ;
Y est choisi dans l'ensemble consistant en O, S et NH ;
R₁ représente alkyle, alcényle, aryle, alkyle substitué, alcényle substitué, aryle substitué, alcoxy ou aryloxy ou est absent lorsque X représente halogène ;
R₂ représente H, alkyle, alcényle, aryle, alkyle substitué, alcényle substitué, aryle substitué, alcoxy ou aryloxy, seulement si X représente N, sinon R₂ est absent ;
R₃ est choisi dans l'ensemble consistant en H, amino, hydroxy, thiol, halogène, nitro, amido, acétyle, alkyle, alcényle, aryle, acétyle substitué, alkyle substitué, alcényle substitué, aryle substitué, alcoxy ou aryloxy ;
R₄ est choisi dans l'ensemble consistant en alkyle, alcényle, aryle, alkyle substitué, alcényle substitué et aryle substitué ; et
dans laquelle ledit marqueur est joint à ladite séquence polymérique monocaténaire par l'intermédiaire d'un site stériquement tolérant reliant R₁, R₂, R₃ ou R₄.

10. Composition comprenant la séquence polymérique monocaténaire de l'une quelconque des revendications 1 à 9 ; et
une séquence de bases polynucléotidique,
dans laquelle une région de ladite séquence polynucléotidique est capable de former un hybride séquence polymérique:polynucléotide avec une région de ladite séquence polymérique monocaténaire, et dans laquelle la stabilité dudit marqueur présent dans ledit hybride séquence polymérique:polynucléotide diffère de la stabilité dudit marqueur en l'absence dudit hybride séquence polymérique:polynucléotide.

11. Composition de la revendication 10, dans laquelle ladite séquence polymérique monocaténaire est hybridée à ladite séquence de bases polynucléotidique pour produire ledit hybride séquence polymérique:polynucléotide.

12. Composition de la revendication 10 ou 11, dans laquelle ladite séquence polymérique monocaténaire et ladite séquence de bases polynucléotidique peuvent former un hybride qui n'est pas parfaitement complémentaire dans lequel ledit marqueur est positionné sur ladite séquence polymérique monocaténaire sur ou près d'une base nucléosidique qui n'est pas parfaitement complémentaire de ladite séquence de bases polynucléotidique, de telle sorte que ledit marqueur soit plus susceptible d'un changement de stabilité que si ladite base nucléosidique était opposée à une partie de ladite séquence de bases polynucléotidique parfaitement complémentaire de ladite séquence de bases polynucléotidique.

13. Composition de la revendication 12, dans laquelle ledit marqueur est positionné sur ladite séquence polymérique monocaténaire en. une position adjacente à un mésappariement présent dans ledit hybride séquence polymérique:polynucléotide.

14. Composition comprenant,
une première séquence polymérique monocaténaire comprenant un marqueur à son extrémité 3' ou 5' ou près de son extrémité 3' ou 5',
une seconde séquence polymérique monocaténaire, et
une séquence de bases polynucléotidique comprenant une région cible,
dans laquelle ladite première séquence polymérique monocaténaire et ladite seconde séquence polymérique monocaténaire sont toutes deux capables de s'hybrider à ladite région cible en des régions adjacentes, de sorte que la stabilité dudit marqueur en présence d'un complexe d'hybridation comprenant ladite première séquence polymérique monocaténaire, ladite seconde séquence polymérique monocaténaire, et ladite région cible, soit supérieure à la stabilité dudit marqueur lorsqu'il est présent sur ladite séquence polymérique monocaténaire qui est monocaténaire ou qui est hybridée à ladite région cible en l'absence de ladite seconde séquence polymérique monocaténaire.

15. Méthode de détection de la présence ou d'une quantité d'une séquence cible d'analyte d'acide nucléique dans un échantillon, comprenant les étapes consistant :
a) à mettre en contact ledit échantillon avec la séquence polymérique monocaténaire de l'une quelconque des revendications 1 à 9, où ladite séquence polymérique monocaténaire peut s'hybrider à ladite séquence cible pour former un complexe sonde:cible comprenant ladite séquence polymérique monocaténaire et ladite séquence cible si ledit analyte est présent dans ledit échantillon, où la stabilité dudit marqueur dans ledit complexe sonde:cible diffère de la stabilité dudit marqueur non présent dans ledit complexe sonde:cible ;
b) à traiter ledit échantillon de façon à provoquer une différence dans la capacité dudit marqueur à être détecté lorsqu'il n'est pas présent dans ledit complexe sonde:cible par rapport au cas où ledit marqueur est présent dans ledit complexe sonde:cible ;
c) à détecter la présence d'un signal issu dudit marqueur présent dans ledit complexe sonde:cible, ou issu dudit marqueur non présent dans ledit complexe sonde:cible, en tant que mesure de la présence ou d'une quantité dudit analyte.

16. Méthode de la revendication 15, dans laquelle ladite séquence polymérique monocaténaire non présente dans ledit complexe sonde:cible est une sonde d'acide nucléique non hybridée, et ladite étape (c) détecte la présence d'un signal issu dudit marqueur présent dans ledit complexe sonde:cible en tant que mesure de la présence ou d'une quantité dudit analyte.

17. Méthode de la revendication 15, dans laquelle ledit marqueur non présent dans ledit complexe sonde:cible peut être présent dans un complexe sonde:non cible comprenant ladite séquence polymérique monocaténaire et un acide nucléique non cible, et ladite étape (c) détecte la présence d'un signal issu dudit marqueur présent dans ledit complexe sonde:cible en tant que mesure de la présence ou d'une quantité dudit analyte.

18. Méthode de la revendication 17, dans laquelle ledit acide nucléique non cible diffère de ladite séquence cible par au moins un nucléotide.

19. Méthode de l'une quelconque des revendications 15 à 18, comprenant en outre la séparation de ladite séquence polymérique monocaténaire dans ledit complexe sonde:cible de ladite séquence polymérique monocaténaire qui n'est pas dans ledit complexe sonde:cible avant ladite étape c).

20. Méthode de détection de la présence ou d'une quantité d'une séquence cible d'analyte d'acide nucléique dans un échantillon comprenant les étapes consistant :
a) à mettre en contact ledit échantillon avec la première et la seconde séquences polymériques monocaténaires de la revendication 14, où ladite première et ladite seconde séquences polymériques monocaténaires peuvent s'hybrider à ladite séquence cible pour former un complexe sonde:cible comprenant ladite première séquence, ladite seconde séquence et ladite séquence cible si ledit analyte est présent dans ledit échantillon, où la stabilité dudit marqueur dans ledit complexe sonde:cible diffère de la stabilité dudit marqueur non présent dans ledit complexe sonde:cible ;
b) à traiter ledit marqueur dans des conditions dans lesquelles la stabilité dudit marqueur diffère dans le complexe sonde:cible de la stabilité dudit marqueur non présent dans le complexe sonde:cible de façon à provoquer une différence dans la capacité dudit marqueur à être détecté lorsqu'il n'est pas présent dans ledit complexe sonde:cible par rapport au cas où ledit marqueur est présent dans ledit complexe sonde:cible ;
c) à détecter la présence d'un signal issu dudit marqueur présent dans ledit complexe sonde:cible, ou issu dudit marqueur non présent dans ledit complexe sonde:cible, en tant que mesure de la présence ou d'une quantité dudit analyte.

21. Méthode de la revendication 20, dans laquelle ladite première séquence polymérique monocaténaire non présente dans ledit complexe sonde:cible est une sonde d'acide nucléique non hybridée, et ladite étape (c) détecte la présence d'un signal issu dudit marqueur présent dans ledit complexe sonde:cible en tant que mesure de la présence ou d'une quantité dudit analyte.

22. Séquence polymérique monocaténaire de l'une quelconque des revendications 1 à 9, ou composition de l'une quelconque des revendications 10 à 14, dans laquelle la stabilité est une résistance à la dégradation.
